# EUROPÄISCHE PATENTSCHRIFT

(11) **EP 1 425 014 B1**
(45) Veröffentlichungstag und Bekanntmachung des Hinweises auf die Patenterteilung: **13.12.2006**
(21) Anmeldenummer: 02797589.5
(22) Anmeldetag: 17.08.2002
(51) Int. Cl.: A61K 31/421, C07D 263/32, C07D 413/12, A61K 31/422, A61P 3/06

(54) **DIARYLCYCLOALKYLDERIVATE, VERFAHREN ZU IHRER HERSTELLUNG UND IHRE VERWENDUNG ALS PPAR-AKTIVATOREN**
DIARYL CYCLOALKYL DERIVATIVES, METHOD FOR PRODUCING THE SAME AND THE USE THEREOF AS PPAR ACTIVATORS
DERIVES DE DIARYLCYCLOALKYLE, LEUR PROCEDE DE PRODUCTION ET LEUR UTILISATION EN TANT QU'ACTIVATEURS DES PPAR

(30) Priorität: 31.08.2001 DE 10142734; 24.05.2002 DE 10223273
(43) Veröffentlichungstag der Anmeldung: 09.06.2004
(73) Patentinhaber: Sanofi-Aventis Deutschland GmbH, 65929 Frankfurt am Main (DE)
(72) Erfinder: GLOMBIK,Heiner, 65719 Hofheim (DE); FALK, Eugen, 60529 Frankfurt (DE); FRICK, Wendelin, 65510 Hünstetten-Beuerbach (DE); KEIL, Stefanie, 65719 Hofheim (DE); SCHÄFER, Hans-Ludwig, 65239 Hochlheim (DE); SCHWINK, Lothar, 35260 stadtallendorf (DE); WENDLER, Wolfgang, D-65618 Selters (DE)
(86) Internationale Anmeldenummer: PCT/EP2002/009221
(87) Internationale Veröffentlichungsnummer: WO 2003/020269

(56) Entgegenhaltungen:
- WO-A-00/64876

## Beschreibung

Die Erfindung betrifft Diarylcycloalkylderivate sowie deren physiologisch verträgliche Salze und physiologisch funktionelle Derivate.

Es sind bereits strukturähnliche Verbindungen zur Behandlung von Hyperlipidämie und Diabetes im Stand der Technik beschrieben (PCT/US/00/11490).

Der Erfindung lag die Aufgabe zugrunde, Verbindungen zur Verfügung zu stellen, die eine therapeutisch verwertbare Triglycerid- senkende Wirkung entfalten mit günstiger Beeinflussung des Lipid-und Kohlenhydratstoffwechsels, besonders bei den Krankheitsbildern der Dyslipidämien, des Diabetes Typ II und des metabolischen Syndroms / Syndrom X. Insbesondere bestand die Aufgabe darin, Verbindungen mit verbesserter Wirkung gegenüber den Verbindungen aus WO 00/64876 zur Verfügung zu stellen. Dies soll insbesonders durch eine Aktivierung des PPARα-Rezeptor erreicht werden.

Die Erfindung betrifft daher Verbindungen der Formel I 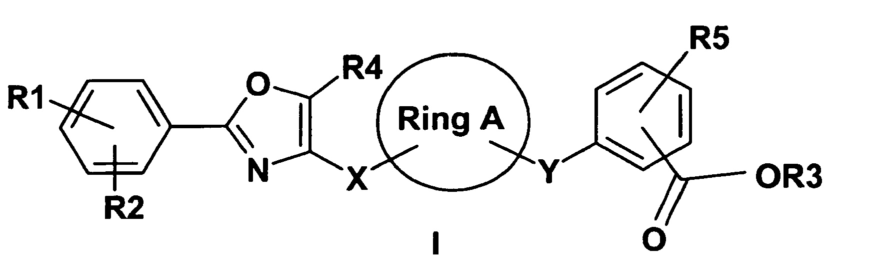 worin bedeuten
- Ring A: (C₃-C₈)-Cycloalkandiyl, (C₃-C₈)-Cycloalkendiyl, wobei in den Cycloalkandiyl- oder Cycloalkendiylringen ein oder mehrere Kohlenstoffatome durch Sauerstoffatome ersetzt sein können;
- R1, R2, R4, R5: unabhängig voneinander H, F, Cl, Br, OH, NO₂, CF₃, OCF₃, (C₁-C₆)-Alkyl, O-(C₁-C₆)-Alkyl;
- R3: H, (C₁-C₆)-Alkyl;
- X: (C₁-C₆)-Alkandiyl, wobei in der Alkandiylgruppe ein oder mehrere Kohlenstoffatome durch Sauerstoffatome ersetzt sein können;
- Y: (C₁-C₆)-Alkandiyl, wobei in der Alkandiylgruppe ein oder mehrere Kohlenstoffatome durch Sauerstoffatome ersetzt sein können;
sowie deren physiologisch verträgliche Salze.

Bevorzugt sind Verbindungen der Formel I, worin bedeuten
- Ring A: (C₃-C₈)-Cycloalkandiyl, (C₃-C₈)-Cycloalkendiyl, wobei in den Cycloalkandiyl- oder Cycloalkendiylringen ein oder mehrere Kohlenstoffatome durch Sauerstoffatome ersetzt sein können;
- R1, R2, R4: unabhängig voneinander H, F, Cl, Br, OH, NO₂, CF₃, OCF₃, (C₁-C₆)-Alkyl, O-(C₁-C₆)-Alkyl;
- R5: (C₁-C₆)-Alkyl;
- R3: H, (C₁-C₆)-Alkyl;
- X: (C₁-C₆)-Alkandiyl, wobei in der Alkandiylgruppe ein oder mehrere Kohlenstoffatome durch Sauerstoffatome ersetzt sind;
- Y: (C₁-C₆)-Alkandiyl, wobei in der Alkandiylgruppe ein oder mehrere Kohlenstoffatome durch Sauerstoffatome ersetzt sein können;
sowie deren physiologisch verträgliche Salze.

Besonders bevorzugt sind Verbindungen der Formel I, worin bedeuten
- Ring A: (C₃-C₈)-Cycloalkandiyl, (C₃-C₈)-Cycloalkendiyl;
- R1, R2: unabhängig voneinander H, F, Cl, Br, OH, NO₂, CF₃, OCF₃, (C₁-C₆)-Alkyl, O-(C₁-C₆)-Alkyl;
- R3: H, (C₁-C₆)-Alkyl;
- X: (C₁-C₆)-Alkandiyl, wobei in der Alkandiylgruppe ein oder mehrere Kohlenstoffatome durch Sauerstoffatome ersetzt sind;
- Y: (C₁-C₆)-Alkandiyl, wobei in der Alkandiylgruppe ein oder mehrere Kohlenstoffatome durch Sauerstoffatome ersetzt sind;
sowie deren physiologisch verträgliche Salze.

Ganz besonders bevorzugt sind Verbindungen der Formel I, mit der Struktur Ia 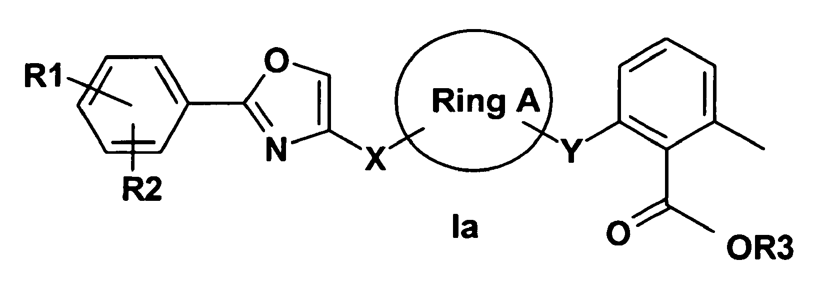 worin bedeuten
- Ring A: Cyclohexandiyl;
- R1, R2: unabhängig voneinander H, F, Cl, Br, OH, NO₂, CF₃, OCF₃, (C₁-C₆)-Alkyl, O-(C₁-C₆)-Alkyl;
- R3: H, (C₁-C₆)-Alkyl;
- X: (C₁-C₆)-Alkandiyl, wobei in der Alkandiylgruppe ein oder mehrere Kohlenstoffatome durch Sauerstoffatome ersetzt sind;
- Y: (C₁-C₆)-Alkandiyl, wobei in der Alkandiylgruppe ein oder mehrere Kohlenstoffatome durch Sauerstoffatome ersetzt sind;
sowie deren physiologisch verträgliche Salze.

Die Erfindung bezieht sich auf Verbindungen der Formel I, in Form ihrer Racemate, racemischen Mischungen und reinen Enantiomere sowie auf ihre Diastereomere und Mischungen davon.

Die Alkylreste in den Substituenten R1, R2, R3, R4 und R5 können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium-und Calciumsalze).

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und, Solvate wie hierin beschrieben.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muß natürlich verträglich sein, in dem Sinne, daß er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, daß die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinalacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wäßrigen oder nicht-wäßrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfaßt, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpreßt oder geformt wird, gegebenenfalls mit einem oder mehreren zusätzlichen Bestandteilen. Gepreßte Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wäßrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor.

Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wäßrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Gegenstand der Erfindung ist weiterhin ein Verfahren zur Herstellung der Verbindungen der allgemeinen Formel I, dadurch gekennzeichnet, daß man die Verbindungen der Formel I so gewinnt, daß gemäß dem folgenden Reaktionsschema vorgegangen wird:

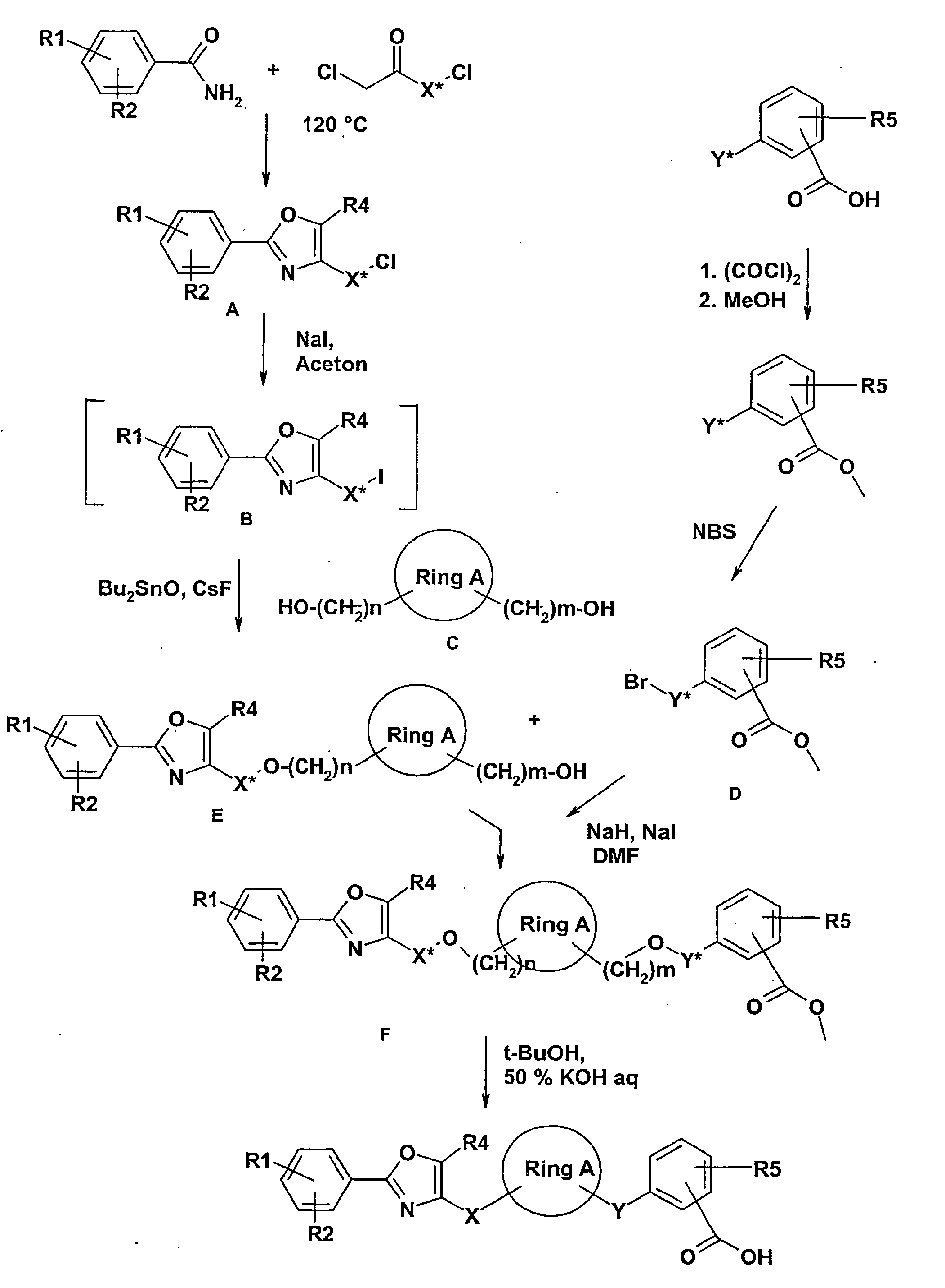

Dazu werden Verbindungen der allgemeinen Formel A, worin R1, R2, R4 und X die oben beschriebenen Bedeutungen haben mit Nal in Aceton unter 12 bis 24 stündigem Erhitzen zum Rückfluss zu einer Verbindung der allgemeinen Formel B umgesetzt.

Die Verbindung der allgemeinen Formel B wird mit einer Verbindung der allgemeinen Formel C, worin n und m jeweils 0-5 bedeuten können, zu einer Verbindung der allgemeinen Formel E umgesetzt, worin R1, R2, R4 m, n und X die oben beschriebenen Bedeutungen haben. Dabei wird a) C in einem inerten Lösemittel wie Dimethylformamid oder Tertahydrofuran mit Natrium-Hydrid bei Raumtemperatur deprotoniert und anschließend mit dem Halogenid bei ca. 70° C umgesetzt oder b) die Komponente C zunächst mit Dibutylzinnoxid in Toluol mehrere Stunden am Wasserabscheider erhitzt und dann unter Zusatz von Dimethylformamid, Cäsiumfluorid und Iodid B durch mehrstündiges Rühren bei Raumtemp. zu E umgesetzt.

Die Verbindung der allgemeinen Formel E wird mit einer Verbindung der allgemeinen Formel D, worin Y die oben beschriebene Bedeutung hat, zu einer Verbindung der allgemeinen Formel F umgesetzt, worin R1, R2, R4, R5 X und Y die oben beschriebenen Bedeutungen haben. Zur Knüpfung einer Etherbindung wird E beispielsweise in einer Mischung aus Dimethylformamid und Tetrahydrofuran mit einr starken Base wie Na-Hydrid bei Raumtemp. deprotoniert und dann mit einer Komponente D vorzugsweise unter Zusatz von Na-Iodid alkyliert.

Die Verbindung der allgemeinen Formel F wird zu Verbindungen der Formel I umgesetzt, indem man die Esterfrunktion beispielsweise durch Erhitzen mit Kaliumhydroxid in einem Alkohol ( Ethanol, tert.Butanol) einer Verseifung unterwirft und die Carbonsäuregruppe der Formel I durch Ansäurern freisetzt. Diese Carbonsäuregruppe kann nach üblichen Methoden zur Gruppe der Formel -(C=O)-OR3, worin R3 die oben beschriebene Bedeutung hat, derivatisiert werden. Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose geeignet.

Die Verbindungen können allein oder in Kombination mit einer oder mehreren weiteren pharmakologisch wirksamen Substanzen verabreicht werden, die beispielsweise eine günstige Wirkungen auf Stoffwechselstörungen haben und die beispielsweise ausgewählt sind aus Antidiabetika, Antiadiposita, blutdrucksenkenden Wirkstoffen und Wirkstoffen zur Behandlung und/oder Prävention von Komplikationen, die von Diabetes verursacht werden oder mit Diabetes assoziiert sind. Als weitere pharmakologisch wirksame Substanzen sind insbesondere geeignet:

Alle Antidiabetika, die in der Roten Liste 2001, Kapitel 12 genannt sind. Sie können mit den erfindungsgemäßen Verbindungen der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.
Antidiabetika umfassen Insulin und Insulinderivate, wie z.B. Lantus^{®} (siehe www.lantus.com) oder HMR 1964, schnell wirkende Insuline (siehe US 6,221,633), GLP-1-Derivate wie z.B. diejenigen die in WO 98/08871 von Novo Nordisk A/S offenbart wurden, sowie oral wirksame hypoglykämische Wirkstoffe.
Die oral wirksamen hypoglykämischen Wirkstoffe umfassen vorzugsweise Sulphonylfharnstoffe, Biguanide, Meglitinide, Oxadiazolidindione, Thiazolidindione, Glukosidase-Inhibitoren, Glukagon-Antagonisten, GLP-1-Agonisten,
Kaliumkanalöffner, wie z.B. diejenigen, die in WO 97/26265 und WO 99/03861 von Novo Nordisk A/S offenbart wurden, Insulin-Sensitizer, Inhibitoren von Leberenzymen, die an der Stimulation der Glukoneogenese und/oder Glykogenolyse beteiligt sind, Modulatoren der Glukoseaufnahme, den Fettstoffwechsel verändernde Verbindungen wie antihyperlipidämische Wirkstoffe und antilipidämische Wirkstoffe, Verbindungen, die die Nahrungsmitteleinnahme verringern, PPAR- und PXR-Agonisten und Wirkstoffe, die auf den ATP-abhängigen Kaliumkanal der Betazellen wirken.

Bei einer Ausführungsform der Erfindung werden die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin verabreicht.

Bei einer Ausführungsform der Erfindung werden die Verbindungen der Formel I in Kombination mit einem Cholesterinresorptionsinhibitor, wie z.B. Ezetimibe, Tiqueside, Pamaqueside, verabreicht.

Bei einer Ausführungsform der Erfindung werden die Verbindungen der Formel I in Kombination mit einem PPAR gamma Agonist, wie z.B. Rosiglitazon, Pioglitazon, JTT-501, GI 262570, verabreicht.

Bei einer Ausführungsform der Erfindung werden die Verbindungen der Formel I in Kombination mit PPAR alpha Agonist, wie z.B. GW 9578, GW 7647, verabreicht.

Bei einer Ausführungsform der Erfindung werden die Verbindungen der Formel I in Kombination mit einem gemischten PPAR alpha/gamma Agonisten, wie z.B. GW 1536, AVE 8042, AVE 8134, AVE 0847, oder wie in PCT/US 11833, PCT/US 11490, DE10142734.4 beschrieben verabreicht.

Bei einer Ausführungsform der Erfindung werden die Verbindungen der Formel I in Kombination mit einem Fibrat, wie z.B. Fenofibrat, Clofibrat, Bezafibrat, verabreicht.

Bei einer Ausführungsform der Erfindung werden die Verbindungen der Formel I in Kombination mit einem MTP-Inhibitor, wie z.B. Implitapide, BMS-201038, R-103757, verabreicht.

Bei einer Ausführungsform der Erfindung werden die Verbindungen der Formel I in Kombination mit Gallensäureresorptionsinhibitor (siehe z.B. US 6,245,744 oder US 6,221,897), wie z:B. HMR 1741, verabreicht.

Bei einer Ausführungsform der Erfindung werden die Verbindungen der Formel I in Kombination mit einem CETP-Inhibitor, wie z.B. JTT-705 , verabreicht.

Bei einer Ausführungsform der Erfindung werden die Verbindungen der Formel I in Kombination mit einem polymeren Gallensäureadsorber, wie z.B. Cholestyramin, Colesevelam, verabreicht.

Bei einer Ausführungsform der Erfindung werden die Verbindungen der Formel I in Kombination mit einem LDL-Rezeptorinducer (siehe US 6,342,512), wie z.B. HMR1171, HMR1586, verabreicht.

Bei einer Ausführungsform der Erfindung werden die Verbindungen der Formel I in Kombination mit einem ACAT-lnhibitor, wie z.B. Avasimibe, verabreicht.

Bei einer Ausführungsform der Erfindung werden die Verbindungen der Formel I in Kombination mit einem Antioxidans, wie z.B. OPC-14117, verabreicht.

Bei einer Ausführungsform der Erfindung werden die Verbindungen der Formel I in Kombination mit einem Lipoprotein-Lipase Inhibitor, wie z.B. NO-1886, verabreicht.

Bei einer Ausführungsform der Erfindung werden die Verbindungen der Formel I in Kombination mit einem ATP-Citrat-Lyase Inhibitor, wie z.B. SB-204990, verabreicht.

Bei einer Ausführungsform der Erfindung werden die Verbindungen der Formel I in Kombination mit einem Squalen Synthetase Inhibitor, wie z.B. BMS-188494, verabreicht.

Bei einer Ausführungsform der Erfindung werden die Verbindungen der Formel I in Kombination mit einem Lipoprotein(a) antagonist, wie z.B. Cl-1027 oder Nicotinsäure, verabreicht.

Bei einer Ausführungsform der Erfindung werden die Verbindungen der Formel I in Kombination mit einem Lipase Inhibitor, wie z.B. Orlistat, verabreicht.

Bei einer Ausführungsform der Erfindung werden die Verbindungen der Formel I in Kombination mit Insulin verabreicht.
Bei einer Ausführungsform werden die Verbindungen der Formel I in Kombination mit einem Sulphonylharnstoff, wie z.B. Tolbutamid, Glibenclamid, Glipizid oder Glimepirid verabreicht.
Bei einer Ausführungsform werden die Verbindungen der Formel I in Kombination mit einem Biguanid, wie z.B. Metformin, verabreicht.

Bei wieder einer Ausführungsform werden die Verbindungen der Formel I in Kombination mit einem Meglitinid, wie z.B. Repaglinid, verabreicht.
Bei einer Ausführungsform werden die Verbindungen der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.
Bei einer Ausführungsform werden die Verbindungen der Formel I in Kombination mit einem α-Glukosidase-Inhibitor, wie z.B. Miglitol oder Acarbose, verabreicht.
Bei einer Ausführungsform werden die Verbindungen der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.
Bei einer Ausführungsform werden die Verbindungen der Formel I in Kombination mit mehr als einer der vorstehend genannten Verbindungen, z.B. in Kombination mit einem Sulphonylharnstoff und Metformin, einem Sulphonylhamstoff und Acarbose, Repaglinid und Metformin, Insulin und einem Sulphonylharnstoff, Insulin und Metformin, Insulin und Troglitazon, Insulin und Lovastatin, etc. verabreicht.

Bei einer weiteren Ausführungsform werden die Verbindungen der Formel I in Kombination mit CART-Modulatoren (siehe "Cocaine-amphetamine-regulated transcript influences energy metabolism, anxiety and gastric emptying in mice" Asakawa, A, et al., M.:Hormone and Metabolic Research (2001), 33(9), 554-558), NPY-Antagonisten z.B. Naphthalin-1-sulfonsäure {4-[(4-amino-quinazolin-2-ylamino)-methyl]-cyclohexylmethyl}- amid; hydrochlorid (CGP 71683A)), MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahyd ro-pyrazolo[4,3-c]pyridin-5-yl)-1-(4-chloro-phenyl)-2-oxo-ethyl]-amid; (WO 01/91752)) Orexin-Antagonisten (z.B. 1-(2-Methyl-benzoxazol-6-yl)-3-[1,5]naphthyridin-4-yl-harnstoff; hydrochloride (SB-334867-A)), H3-Agonisten (3-Cyclohexyl-1-(4,4-dimethyl-1,4,6,7-tetrahydro-imidazo[4,5-c]pyridin-5-yl)-propan-1- on Oxalsäuresalz (WO 00 / 63208)); TNF-Agonisten, CRF-Antagonisten (z.B. [2-Methyl-9-(2,4,6-trimethyl-phenyl)-9H-1,3,9-triaza-fluoren-4-yl]-dipropyl-amin (WO 00/66585)), CRF BP-Antagonisten (z.B. Urocortin), Urocortin-Agonisten, β3-Agonisten (z.B. 1-(4-Chloro-3-methanesulfonylmethyl-phenyl)-2-[2-(2,3-dimethyl-1 H-indol-6-yloxy)-ethylamino]-ethanol; hydrochloride (WO 01/83451)), MSH (Melanocyt-stimulierendes Hormon)-Agonisten, CCK-A Agonisten (z.B. {2-[4-(4-Chloro-2,5-dimethoxy-phenyl)-5-(2-cyclohexyl-ethyl)-thiazol-2-ylcarbamoyl]-5,7- dimethyl-indol-1-yl}-acetic acid Trifluoressigsäuresalz (WO 99/15525)); Serotonin-Wiederaufnahme-Inhibitoren (z.B. Dexfenfluramine), gemischte Sertonin- und noradrenerge Verbindungen (z.B. WO 00/71549), 5HT-Agonisten z.B. 1-(3-Ethyl-benzofuran-7-yl)-piperazin Oxalsäuresalz (WO 01/09111), Bombesin-Agonisten, Galanin-Antagonisten, Wachstumshormon (z.B. humanes Wachstumshormon), Wachstumshormon freisetzende Verbindungen (6-Benzyloxy-1- (2-diisopropylamino-ethylcarbamoyl)-3,4-dihydro-1 H-isoquinoline-2-carboxylic acid tert-butyl ester (WO 01/85695)), TRH-Agonisten (siehe z.B. EP 0 462 884) entkoppelnde Protein 2- oder 3-Modulatoren, Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881),

DA-Agonisten (Bromocriptin, Doprexin), Lipase/Amylase-Inhibitoren (z.B. WO 00/40569), PPAR-Modulatoren (z.B. WO 00/78312), RXR-Modulatoren oder TR,β-Agonisten verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphatamin oder Amphetamin.
Bei einer Ausführungsform ist der weitere Wirkstoff Fenfluramin oder Dexfenfluramin. Bei noch einer Ausführungsform ist der weitere Wirkstoff Sibutramin.
Bei einer Ausführungsform ist der weitere Wirkstoff Orlistat.
Bei einer Ausführungsform ist der weitere Wirkstoff Mazindol oder Phentermin.

Bei einer Ausführungsform werden die Verbindungen der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird. 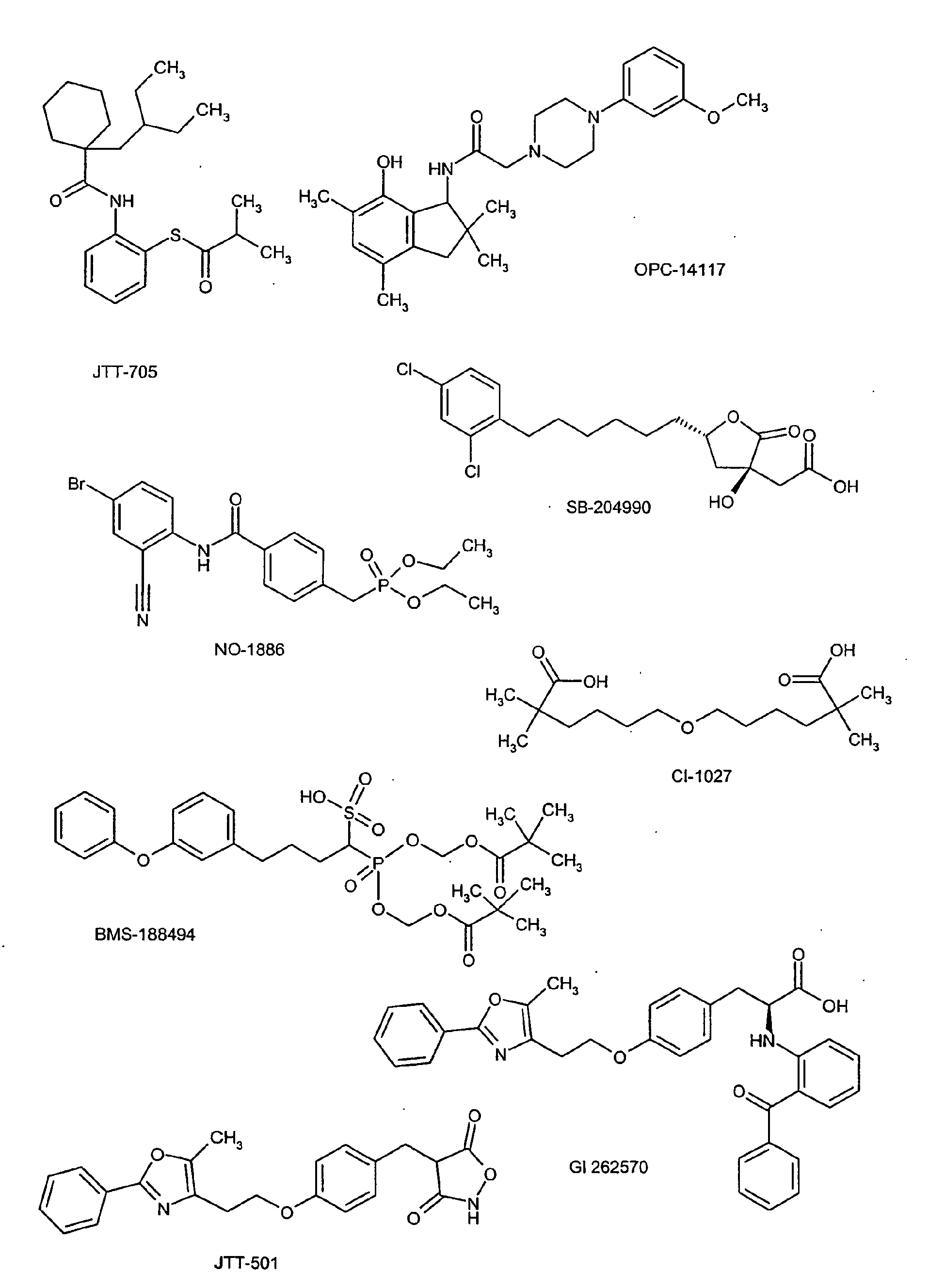 Diese Erfindung bezieht sich weiterhin auf die Verwendung von Verbindungen der Formel I und ihren pharmazeutischen Zusammensetzungen als PPAR-Liganden-Rezeptor-Binder. Die erfindungsgemäßen PPAR-Liganden-Rezeptor-Binder eignen sich als Agonisten oder Antagonisten des PPAR-Rezeptors.
Peroxisom-Proliferator-aktivierte Rezeptoren (PPAR) können in die drei Subtypen PPARα, PPARδ und PPARγ unterteilt werden. Diese werden von verschiedenen Genen codiert (Motojima, Cell Structure and Function, 18:267-277, 1993). Darüber hinaus gibt es zwei Isotope von PPARγ, PPARγ₁ und γ₂. Diese beiden Proteine unterscheiden sich in 30 NH₂-terminalen Aminosäuren und sind das Ergebnis eines alternativen Einsatzes von Promotoren und einer differenziellen mRNA-Spleißung (Vidal-Puig, Jiminez, Linan, Lowell, Hamann, Hu, Spiegelman, Flier, Moller, J. Clin. Invest., 97:2553-2561, 1996).
Bei PPAR-modulierten biologischen Prozessen handelt es sich um solche Prozesse, die von Rezeptoren oder Kombinationen von Rezeptoren moduliert werden, die auf die in diesem Patent beschriebenen PPAR-Rezeptor-Liganden ansprechen. Diese Prozesse umfassen beispielsweise den Plasmalipidtransport und den Fettsäurekatabolismus, die Regulierung von Insulinempfindlichkeit und Blutzuckerspiegeln, die beteiligt sind an Hypoglykämie/Hyperinsulinismus (die z.B. bedingt sind durch Funktionsstörungen der Pankreas-Betazellen, insulinsezernierende Tumoren und/oder Autoimmunhypoglykämie infolge von Autoantikörpern gegen Insulin, den Insulinrezeptor, oder Autoantikörper, die eine stimulierende Wirkung auf Pankreas-Betazellen haben), Makrophagen-Differenzierung, die zur Bildung atherosklerotischer Plaques, zu entzündlichen Reaktionen, Karzinogenese, Hyperplasie oder Adipozyten-Differenzierung führt.
Adipositas ist eine übermäßige Ansammlung von Fettgewebe. Jüngste Arbeiten auf diesem Gebiet haben aufgezeigt, dass PPARγ eine zentrale Rolle bei der Genexpression und Differenzierung von Adipozyten spielt. Übermäßiges Fettgewebe ist assoziiert mit der Entwicklung schwerer Erkrankungen wie beipielsweise nicht-insulinpflichtiger Diabetes mellitus (NIDDM), Hypertonie, Erkrankungen der Koronararterien, Hyperlipidämie, Adipositas und bestimmte maligne Krankheitsbilder.

Die Adipozyten können sich durch die Bildung von Tumornekrosefaktor α (TNFα) und anderen Molekülen auch auf die Glukosehomeostase auswirken.
Nicht-insulinpflichtiger Diabetes mellitus (NIDDM) oder Typ-II-Diabetes ist die häufigere Form von Diabetes. An dieser Form der Krankheit leiden etwa 90-95% der Hyperglykämie-Patienten. Bei NIDDM liegen anscheinend eine Reduzierung der Masse der Pankreas-Betazellen, mehrere verschiedene Störungen der Insulinsekretion oder eine reduzierte Insulinempfindlichkeit des Gewebes vor. Die Symptome dieser Form von Diabetes umfassen Müdigkeit, häufiges Wasserlassen, Durst, verschwommenes Sehen, häufige Infektionen und langsames Heilen von Wunden, diabetische Nervenschädigungen und Nierenerkrankungen.
Resistenz gegen die metabolischen Wirkungen von Insulin ist eines der Hauptmerkmale von nicht-insulinpflichtigem Diabetes (NIDDM). Insulinresistenz ist gekennzeichnet durch eine beeinträchtigte Aufnahme und Umsetzung von Glukose in insulinempfindlichen Zielorganen wie beispielsweise Adipozyten und Skelettmuskeln, sowie durch eine beeinträchtigte Hemmung der hepatischen Glukoneogenese. Der funktionelle Insulinmangel und die fehlende Unterdrückung der hepatischen Glukoneogenese durch Insulin führt zu Hyperglykämie im nüchternen Zustand. Die Pankreas-Betazellen kompensieren die Insulinresistenz, indem sie verstärkt Insulin sezernieren. Doch die Betazellen können diese hohe Insulinbildung nicht aufrechterhalten, so dass die Glukose-induzierte Insulinsekretion zurückgeht und es zu einer Verschlechterung der Glukosehomeostase und schließlich zur Entwicklung eines manifesten Diabetes kommt.
Hyperinsulinämie steht ebenfalls in Zusammenhang mit Insulinresistenz, Hypertriglyceridämie und erhöhten Plasmakonzentrationen von Lipoproteinen niedriger Dichte. Der Zusammenhang von Insulinresistenz und Hyperinsulinämie mit diesen Stoffwechselstörungen wurde "Syndrom X" genannt und wird stark mit einem erhöten Risiko von Hypertonie und Erkrankungen der Koronararterien assoziiert.
Metformin ist dem Fachmann zur Behandlung von Diabetes beim Menschen bekannt (US-Patent Nr. 3,174,901). Metformin bewirkt primär eine reduzierte Glukosebildung in der Leber. Troglitazon^{®} wirkt bekanntlich primär auf die Verbesserung der Fähigkeit der Skelettmuskeln, auf Insulin zu reagieren und Glukose aufzunehmen. Es ist bekannt, dass eine Kombinationstherapie von Metformin und Troglitazon zur Behandlung von Störungen eingesetzt werden kann, die mit Diabetes einhergehen (DDT 3:79-88, 1998).
Es wurde beobachtet, dass PPARγ-Aktivatoren, insbesondere Troglitazon^{®}, bei Liposarkomen (Fett-Tumoren) Krebsgewebe in normale Zellen umwandeln (PNAS 96:3951-3956, 1999). Ferner wurde vermutet, dass PPARγ-Aktivatoren zur Behandlung von Brust- und Darmkrebs nützlich sein könnten (PNAS 95:8806-8811, 1998, Nature Medicine 4:1046-1052,1998).
Darüber hinaus wurden PPARγ-Aktivatoren wie beispielsweise Troglitazon^{®} auch zur Behandlung des polyzystischen Ovarialsyndroms (PCO) eingesetzt. Dieses bei Frauen auftretende Syndrom ist durch chronische Anovulation und Hyperandrogenismus gekennzeichnet. Bei Frauen mit diesem Syndrom liegen häufig auch Insulinresistenz und ein erhöhtes Risiko der Entwicklung von nicht-insulinpflichtigem Diabetes mellitus vor (Dunaif, Scott, Finegood, Quintana, Whitcomb, J. Clin. Endocrinol. Metab., 81:3299,1996).
Ferner wurde kürzlich entdeckt, dass PPARγ-Aktivatoren die Bildung von Progesteron steigern und die Steroidgenese in Granulosa-Zellkulturen hemmen und sich daher zur Behandlung des Klimakteriums eignen können (US-Patent Nr. 5,814,647 Urban et al., 29. September 1998; B. Lorke et al., Journal of Endocrinology, 159, 429-39, 1998). Klimakterium ist definiert als das Syndrom der endokrinen, somatischen und psychologischen Veränderungen, die zum Ende der fortpflanzungsfähigen Phase von Frauen auftreten.
Peroxisome sind Zellorganellen, die an der Kontrolle von Redox-Potenzial und oxidativem Stress von Zellen beteiligt sind, indem sie eine Vielzahl von Substraten wie beispielsweise Wasserstoffperoxid metabolisieren. Es gibt eine Reihe von Störungen, die mit oxidativem Stress assoziiert sind. So gehen beispielsweise entzündliche Reaktionen auf Gewebeverletzungen, die Pathogenese von Emphysemen, Ischämieassoziierte Organschädigungen (Schock), Doxorubicin-induzierte Herzschädigungen, Arzneimittel-induzierte Hepatotoxizität, Atherosklerose und durch Hyperoxie bedingte Lungenschädigungen jeweils mit der Bildung reaktiver Sauerstoff-Spezies und einer Veränderung der Reduktionsfähigkeit der Zelle einher. Daher wird erwogen, dass PPARα-Aktivatoren unter anderem das Redox-Potenzial und den oxidativen Stress in Zellen regulieren und zur Behandlung dieser Störungen nützlich sein könnten (Poynter et al., J. Biol. Chem. 273, 32833-41, 1998).
Es wurde ebenfalls entdeckt, dass PPARα-Agonisten die NF_{K}B-mediierte Transkription hemmen und dadurch verschiedene Entzündungsreaktionen modulieren, wie etwa die Enzympfade der induzierbaren Stickoxid-Synthase (NOS) und Cyclooxygenase-2 (COX-2) (Pineda-Torra, I. et al., 1999, Curr. Opinion in Lipidology, 10, 151-9) und daher für therapeutische Eingriffe bei einer großen Vielfalt von Entzündungskrankheiten und anderen pathologischen Zuständen eingesetzt werden können (Colville-Nash et al., Journal of Immunology, 161, 978-84, 1998; Staels et al, Nature, 393, 790-3, 1998).
Peroxisom-Proliferatoren aktivieren PPAR, die wiederum als Transkriptionsfaktoren wirken und Differenzierung, Zellwachstum und Proliferation von Peroxisomen verursachen. Es wird auch vermutet, dass PPAR-Aktivatoren eine Rolle bei Hyperplasie und Carcinogenese spielen und die enzymatischen Fähigkeiten von Tierzellen wie beispielsweise Nagerzellen verändern, doch diese PPAR-Aktivatoren scheinen nur minimale negative Auswirkungen auf menschliche Zellen zu haben (Green, Biochem. Pharm. 43(3):393, 1992). Die Aktivierung von PPAR führt zu einem raschen Anstieg von Gammaglutamyltranspeptidase und -katalase.
PPARα wird durch eine Reihe von Fettsäuren mittlerer Länge und langkettigen Fettsäuren aktiviert und ist an der Stimulierung der β-Oxidation von Fettsäuren in Geweben wie Leber, Herz, Skelettmuskel und braunes Fettgewebe beteiligt (Issemann und Green, ibid.; Beck et al., Proc. R. Soc. Lond. 247:83-87, 1992; Gottlicher et al., Proc. Natl. Acad. Sci. USA 89:4653-4657, 1992). Pharmakologische PPARα-Aktivatoren wie beispielsweise Fenofibrat, Clofibrat, Gemfibrozil und Bezafibrat sind ebenfalls an der erheblichen Reduzierung von Plasmatriglyceriden sowie einer mäßigen Reduzierung von LDL-Cholesterin beteiligt, und sie werden insbesondere zur Behandlung von Hypertriglyceridämie, Hyperlipidämie und Adipositas eingesetzt. PPARα ist bekanntlich auch an entzündlichen Störungen beteiligt (Schoonjans, K., Current Opinion in Lipidology, 8, 159-66, 1997).
Der menschliche nukleäre Rezeptor PPARδ wurde aus einer cDNA-Bibliothek menschlicher Osteosarkomzellen kloniert und wird bei A. Schmidt et al., Molecular Endocrinology, 6:1634-1641 (1992) vollständig beschrieben. Der Inhalt dieser Ausführungen wird durch Bezugnahme in diese Patentschrift aufgenommen. Es sei darauf hingewiesen, dass PPARδ in der Literatur auch als PPARβ und als NUC1 bezeichnet wird, wobei sich jeder dieser Namen auf denselben Rezeptor bezieht. So wird der Rezeptor beispielsweise bei A. Schmidt et al., Molecular Endocrinology, 6:1634-1641, 1992 als NUC1 bezeichnet. PPARδ wird sowohl in embryonalen als auch in adulten Geweben festgestellt. Es wurde berichtet, dass dieser Rezeptor an der Regulierung der Expression einiger fettspezifischer Gene beteiligt ist und eine Rolle im Prozess der Adipogenese spielt (Amri, E. et al., J. Biol. Chem. 270, 2367-71, 1995). Man weiß, dass atherosklerotische Erkrankungen durch eine Reihe von Faktoren verursacht werden wie beispielsweise Hypertonie, Diabetes, geringe Spiegel von Lipoproteinen hoher Dichte (HDL) und hohe Spiegel von Lipoproteinen niedriger Dichte (LDL). Zusätzlich zur Reduzierung der Risiken durch Effekte auf die Konzentration der Plasmalipide und andere Risikofaktoren haben PPARα-Agonisten direkte atheroprotektive Wirkungen (Frick, M.H. et al., 1997, Circulation 96:2137-2143, de Faire et al., 1997, Cardiovasc. Drugs Ther. 11 Suppl. 1:257-63).
Kürzlich wurde festgestellt, dass PPARδ-Agonisten nützlich sind, um HDL-Spiegel zu erhöhen und sich daher zur Behandlung atherosklerotischer Erkrankungen eignen (Leibowitz et al., WO/9728149). Atherosklerotische Erkrankungen umfassen Gefäßkrankheiten, koronare Herzkrankheit, zerebrovaskuläre Erkrankungen und Erkrankungen der peripheren Gefäße. Koronare Herzkrankheit umfasst Tod durch koronare Herzkrankheit, Myokardinfarkt und koronare Revaskularisierung. Zerebrovaskuläre Erkrankungen umfassen ischämische oder hämorrhagische Infarkte und transiente ischämische Anfälle.
PPARγ-Subtypen sind an der Aktivierung der Adipozyten-Differenzierung beteiligt und spielen keine Rolle bei der Stimulierung der Peroxisomproliferation in der Leber. Die Aktivierung von PPARγ ist an der Adipozyten-Differenzierung durch die Aktivierung der Adipozyten-spezifischen Genexpression beteiligt (Lehmann, Moore, Smith-Oliver, Wilkison, Willson, Kliewer, J. Biol. Chem., 270:12953-12956, 1995). Die DNA-Sequenzen der PPARγ-Subtypen sind bei Elbrecht et al., BBRC 224; 431-437 (1996) beschrieben. Obwohl Peroxisom-Proliferatoren einschließlich Fibraten und Fettsäuren die transkriptorische Aktivität von PPARs aktivieren, wurden nur Prostaglandin J₂-Derivate wie der Arachidonsäure-Metabolit 15-Deoxy-Delta¹², 14-Prostaglandin J₂ (15d-PGJ₂) als natürliche Liganden identifiziert, die spezifisch für den PPARγ-Subtyp sind, der auch an Thiazolidindione bindet. Dieses Prostaglandin aktiviert die PPARγ-abhängige Adipogenese, aktiviert PPARα aber nur in hohen Konzentrationen (Formann, Tontonoz, Chen, Brun, Spiegelman, Evans, Cell, 83:803-812, 1995; Kliewer, Lenhard, Wilson, Patel, Morris, Lehmann, Cell, 83:813-819, 1995). Dies ist ein weiterer Hinweis darauf, dass die Subtypen der PPAR-Familie sich in ihrer pharmakologischen Reaktion auf Liganden unterscheiden.
Daraus ergibt sich, dass Verbindungen, die PPARα oder sowohl PPARα als auch PPARγ aktivieren, wirkungsvolle hypotriglyceridämische Arzneimittel sein müssten, die zur Behandlung von mit Atherosklerose assoziierter Dislipidämie, nicht-insulinpflichtigem Diabetes mellitus, Syndrom X (Staels, B. et al., Curr. Pharm. Des., 3 (1), 1-4 (1997)) und familiärer kombinierter Hyperlipidämie (FCH) eingesetzt werden können. Syndrom X ist das Syndrom, das durch ein erstes insulinresistentes Stadium charakterisiert ist, das Hyperinsulinämie, Dyslipidämie und eine beeinträchtigte Glukosetoleranz bewirkt und zu nicht-insulinpflichtigem Diabetes mellitus (Typ II-Diabetes) progredieren kann, der durch Hyperglykämie gekennzeichnet ist. FCH ist durch Hypercholesterinämie und Hypertriglyceridämie bei demselben Patienten und in derselben Familie gekennzeichnet.
Die vorliegende Erfindung betrifft Verbindungen der Formel I, die sich zur Modulierung von PPAR-Rezeptoren eignen, sowie eine Reihe anderer damit verbundener pharmazeutischer Anwendungen.
Die Verbindungen der Formel I eignen sich insbesonders zur Behandlung von Dyslipidämie, Insulinresistenz, Typ I und Typ II Diabetes, Störungen der Glucose-Toleranz, Syndrom X, Obesitas, Essstörungen, Thrombosen, Entzündungen, Cardiomyopathie sowie zum Beta-Zellen Schutz und Fettsäure-Oxidationsschutz (siehe z.B. Jean-Charles Fruchart, Bart Staels and Patrick Duriez:
PPARS, Metabolic Disease and Atherrosclerosis, Pharmacological Research, Vol. 44, No. 5, 2001; Sander Kersten, Beatrice Desvergne & Walter Wahli: Roles of PPARs in health and disease, NATURE, VOL 405 ,25 MAY 2000 ; Ines Pineda Torra, Giulia Chinetti, Caroline Duval, Jean-Charles Fruchart and Bart Staels:
Peroxisome proliferator-activated receptors: from transcriptional
control to clinical practice, Curr Opin Lipidol 12: 2001, 245-254).
Die Wirksamkeit der Verbindungen wurde wie folgt getestet:

Für die Analyse der Wirkstärke von Substanzen, die an humanes PPARalpha binden und es in agonistischer Weise aktivieren, wird eine stabil transfizierte HEK-Zellinie (HEK= human embryo kidney) benutzt, die hier als "PPARalpha-Reporterzellinie" bezeichnet wird.
Die Aktivität von PPARalpha-Agonisten wird in einem 3-Tagestest bestimmt, der nachfolgend beschrieben ist:

Die PPARalpha-Reporterzellinie wird bis zu einer 80 %igen Konfluenz in DMEM-Medium (# 41965-039, Life Technologies) kultiviert, das mit folgenden Zusätzen versehen ist: 10% cs-FKS (fötales Kälberserum, #SH-30068.03, Hyclone), Antibiotika (0,5 mg/ml Zeozin [#R250-01, Invitrogen], 0,5 mg/ml G418 [#10131-019, Life Technologies], 1 % Penicillin-Streptomycin- Lösung [#15140-031, Life Technologies]) und 2 mM L-Glutamin (#25030-032, Life Technologies). Die Kultivierung erfolgt in Standard-Zellkulturflaschen (# 33111, Becton Dickinson) in einem Zellkulturbrutschrank bei 37°C und 5% CO₂. Die zu 80% konfluenten Zellen werden einmal mit 30 ml PBS gewaschen (#14190-094, Life Technologies), mit 2 ml Trypsinlösung (#25300-054, Life Technologies) für 2 min bei 37°C behandelt, in 5 ml des oben beschriebenen Mediums aufgenommen und in einem Zellzählgerät gezählt. Nach der Verdünnung auf 500.000 Zellen/ml werden jeweils 100.000 Zellen pro Loch einer 96 Loch-Mikrotiterplatte mit klarem Plastikboden (#3610, Corning Costar) ausgesät. Die Platten werden für 24 h in einem Zellkulturbrutschrank bei 37°C und 5% CO₂ inkubiert.

Zu testende PPARalpha-Agonisten werden in einer Konzentration von 10 mM in DMSO gelöst. Diese Stocklösung wird in phenolrot-freiem DMEM Medium (#21063-029, Life Technologies) verdünnt, das mit 5% of cs-FKS (#SH-30068.03, Hyclone), 2 mM L-Glutamin (#25030-032, Life Technologies) und den bereits unter dem Punkt "Aussaat der Zellen" beschriebenen Antibiotika (Zeozin, G418, Penicillin und Streptomycin) versetzt war.
Üblicherweise werden Testsubstanzen in 11 verschiedenen Konzentrationen getestet (10 µM; 3.3 µM; 1 µM; 0.33 µM; 0,1 µM; 0,033 µM; 0,01 µM; 0,0033 µM; 0,001 µM; 0,00033 µM; und 0,0001 µM). Potentere Verbindungen werden in Konzentrationsbereichen von 1 µM bis 10 pM bzw. 100 nM bis 1 pM geprüft.
Das Medium der an Tag 1 ausgesäten PPARalpha-Reporterzellinie wird vollständig aus jedem Loch abgesaugt und die in Medium verdünnten Testsubstanzen sofort zu den Zellen zugegeben. Die Verdünnung und Zugabe der Substanzen kann mit einem Roboter erfolgen (Beckman Biomek 2000). Das Endvolumen der in Medium verdünnten Testsubstanzen beträgt 100 µl pro Loch einer 96 Lochplatte. Die DMSO-Konzentration in dem Assay ist immer unter 0.1 % v/v, um zelltoxische Effekte des Lösungsmittels zu vermeiden.
Jede Platte wird mit einem Standard PPARalpha-Agonisten belegt, der ebenfalls in 11 verschiedenen Konzentrationen verdünnt wird, um die Funktionsfähigkeit des Assays in jeder Einzelplatte nachzuweisen. Die Testplatten werden für 24h in einem Brutschrank bei 37°C und 5% CO₂ inkubiert.

Die mit den Testsubstanzen behandelten PPARalpha-Reporterzellen werden aus dem Brutschrank entnommen und für 1h bei -20°C eingefroren, um die Zelllyse zu verbessern. Nach dem Auftauen der Platten, das über mindestens 30 min. bei Raumtemperatur erfolgt, werden 50 µl Puffer 1 (Luc-Screen kit #LS1000, PE Biosystems Tropix) zu jedem Loch zupipettiert und die Platten im Anschluß daran in ein Lumineszenzmeßgerät mit Pipettiereinheit (Luminoscan Ascent, LabSystems) überführt. Die Luziferasereaktion wird in dem Meßgerät durch Zupipettieren von je 50 µl Puffer 2 (Luc-Screen kit #LS1000, PE Biosystems Tropix) zu jedem Loch der 96 Lochplatte gestartet. Die Zugabe des Puffers in jedes einzelne Loch erfolgt in definierten und gleichen Zeitintervallen nach den Angaben des Geräteherstellers (LabSystems). Alle Proben werden exakt 16 min. nach Zugabe von Puffer 2 gemessen. Die Meßzeit beträgt 10 sec. pro Probe.

Die Rohdaten des Lumineszenzmeßgerätes werden in ein Microsoft Excel-File transferiert. Dosis-Wirkungskurven, sowie EC₅₀-Werte werden mit dem Programm XL.Fit nach Vorgabe des Herstellers (IDBS) berechnet.

Die Ergebnisse für die Aktivität der erfindungsgemäßen Verbindungen der Formel I sind in der folgenden Tabelle I angegeben:

**Tabelle I**

| **Beispiel Nr.** | **EC50 PPARalpha [nM]** |
|---|---|
| I | 1 |
| II | 0.3 |
| IV | 0.3 |
| VII | 4 |
| X | 0.5 |
| XIX | 16 |
| XXIV | 0.9 |
| XXV | 13 |
| XXVIII | 14 |
| XXIX | 32 |
| XXXII | 0.97 |
| XXXIV | 0.82 |
| XXXVI | 0.62 |
| XXXVIII | 0.57 |
| XLI | 0.6 |
| XLIII | 0.58 |
| XLIV | 0.93 |
| XLV | 10 |
| XLVI | 0.56 |
| XLVII | 1.1 |

Aus der Tabelle I ist ersichtlich, daß die erfindungsgemäßen Verbindungen der Formel 5 I den PPARα-Rezeptor aktivieren und damit analog zu klinisch verwendeten Fibraten im Organismus eine Triglyceridsenkung bewirken (siehe z.B. J.-Ch. Fruchard et al.,: PPARS, Metabolic Disease and Atherosclerosis,
Pharmacological Research, Vol. 44, No. 5, 2001; S. Kersten et al.: Roles of PPARs in health and disease, NATURE, VOL 405 ,25 MAY 2000 ;I. Pineda et al.:
Peroxisome proliferator-activated receptors: from transcriptional
control to clinical practice,Curr Opin Lipidol 12: 2001 , 245-254).

Die nachfolgend aufgeführten Beispiele dienen zur Erläuterung der Erfindung, ohne diese jedoch einzuschränken. Die gemessenen Fest-, bzw. Zersetzungspunkte (Fp.) wurden nicht korrigiert und sind generell von der Aufheizgeschwindigkeit abhängig.

### Beispiel I

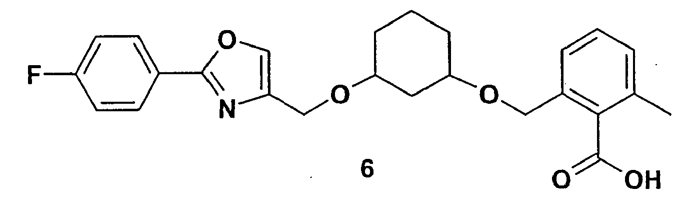

### 3-[2-(4-Fluorphenyl)-oxazol-4-ylmethoxy]-cyclohexanol 3

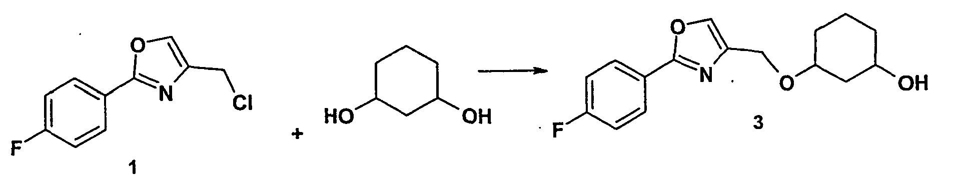

In eine Mischung von 50 ml Dimethylformamid und 50 ml Tetrahydrofuran gibt man unter Eiskühlung zunächst 2.25 g 80-proz. Natriumhydrid-Suspension und dann 5.8g 1,3-Cyclohexandiol. Man rührt 3 Std. bei ca. 25°C nach. Dann gibt man 10.5 g 4-Chlormethyl-2-(4-fluorophenyl)-oxazol (1) dazu, erwärmt auf 70°C und kontrolliert durch Dünnschichtchromatographie. Nach beendeter Umsetzung wird auf Eiswasser gegossen und mit Ethylacetat extrahiert. Nach Abtrennung wird die organische Phase getrocknet, eingeengt und der Rückstand an Kieselgel durch Flash-Chromatographie gereinigt (Ethylacetat/n-Heptan =1:1). Man erhält den Alkohol **3** als Öl. C₁₆H₁₈FNO₃ (291.33) MS(ESI): 292 (M+H⁺)

### 2-{3-[2-(4-Fluor-phenyl)-oxazol-4-ylmethoxy]-cyclohexyloxy}-6-methyl-benzoesäuremethylester 5

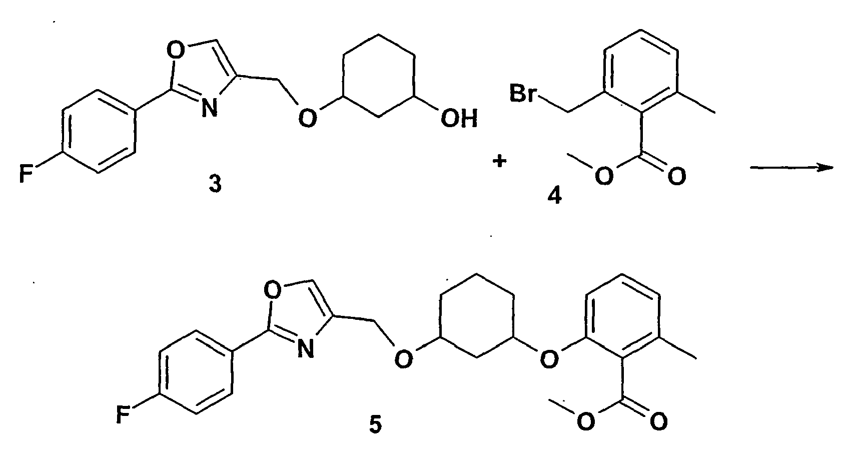

In eine Mischung von 10 ml Dimethylformamid und 20 ml Tetrahydrofuran werden unter Eiskühlung 0.3 g Natriumhydrid-Suspension (80%) eingetragen. Anschließend gibt man 1g Alkohol **3** in 5 ml Tetrahydrofuran dazu und rührt **1** Std. bei Raumtemp. Danach bibt man 0.8 g Bromid **4** dazu und rührt unter DC-Kontrolle bei Raumtemp. 3-5 Std. bis zu weitgehender Umsetzung. Man gießt auf Eiswasser, extrahiert mehrfach mit Ethylacetat, wäscht die organische Phase mit wenig Wasser, trocknet über Natriumsulfat, engt i.Vak. ein und reinigt den Rückstand durch Chromatographie an Kieselgel (Ethylacetat:n-Heptan = 1:2). Man erhält den Methylester **5** als Öl. C₂₆H₂₈FNO₅ (453.52) MS(ESI): 454 (M + H⁺).

### 2-{3-[2-(4-Fluorphenyl)-oxazol-4-ylmethoxy]-cyclohexyloxy}-6-methyl -benzoesäure 6

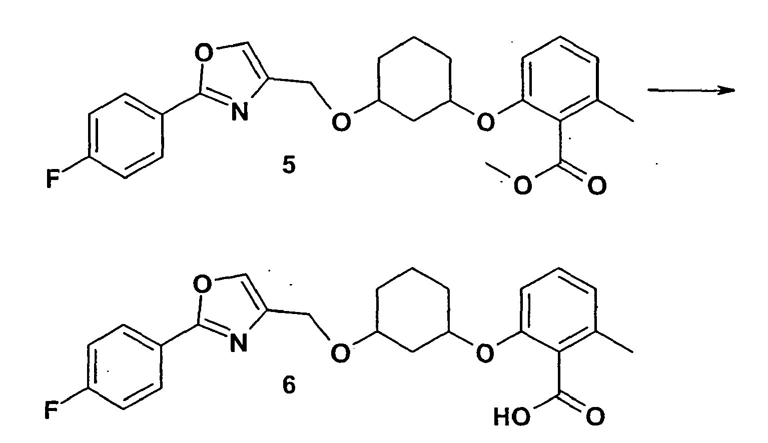

2g Ester 5 werden in 150 ml tert.Butanol und 24 ml 50proz. Kalilauge 6 Std. zum Rückfluss erhitzt. Man entfernt 4/5 des Butanols i.Vak., verdünnt mit Wasser und säuert unter Eiskühlung an. Man extrahiert das Produkt mit Dichlormethan, trocknet über Natriumsulfat, engt i.Vak. ein und erhält durch Filtration des Rückstands über Kieselgel (CH₂Cl₂ / MeOH = 20:1) die Säure **6** C₂₅H₂₆FNO₅ (432.42) MS(ESI): 433 (M + H⁺).

### Beispiel II

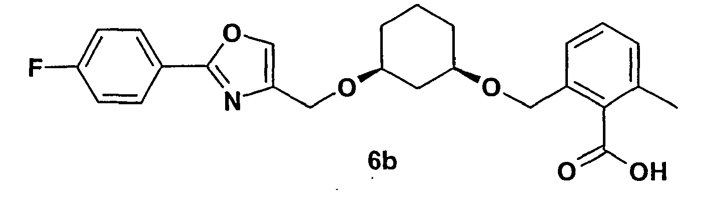

### 2-(4-Fluorphenyl)-4-iodmethyl-oxazol 2

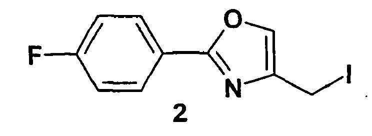

31 g (123 mmol) p-Fluorbenzamid und 33 g (123 mmol) 1,3-Dichloraceton werden bei 120 °C ohne Lösungsmittel 2 Stunden gerührt. Nach dem Abkühlen auf Raumtemperatur wird mit 250 ml Etylacetat angelöst. Diese Lösung verdünnt man mit 400 ml n-Heptan und wäscht 3 mal mit gesättigter NaCl-Lösung. Die organische Phase wird über 250 ml Kieselgel filtriert und mit 200 ml n-Heptan/Ethylacetat (4:1) nachgewaschen. Nach dem Abdestillieren des Lösungsmittels erhält man 4-Chlormethyl-2-(4-fluorphenyl)-oxazol 1 als Rohprodukt. Dieses wird in 650 ml Aceton gelöst und dann werden 90 g Nal zugegeben. Anschließend wird 16 Stunden zum Rückfluß erhitzt, dann das Lösungsmittel weitgehend entfernt, der feste Rückstand in 200 ml n-Heptan/Ethylacetat (1:1) suspendiert und über 200 ml Kieselgel filtriert. Der Niederschlag wird noch mit 500 ml n-Heptan/Ethylacetat (1:1) nachgewaschen und die organische Phase eingeengt. Beim Einengen beginnt die Kristallisation des Iodides **2** als weißes Kristallisat. DC n-Heptan/Ethylacetat (6:1). R_{f} = 0.4 für **2** und R_{f} = 0.35 für **1.** C₁₀H₇FlNO (303.08) MS(ESI): 304 (M + H⁺).

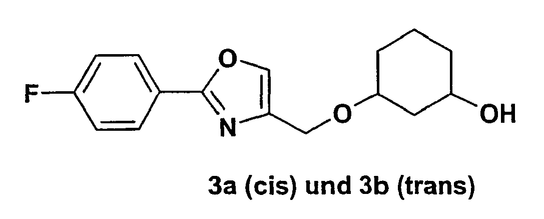

10.8 g (93.1 mmol) cis/trans-1,3-Cyclohexandiol und 15.4 g (61.8 mmol) Dibutylzinnoxid werden in 800 ml Toluol 5 Stunden am Wasserabscheider erhitzt. Nach dem Abdestillieren von 400 ml Toluol läßt man auf Raumtemperatur abkühlen und gibt nacheinander 280 ml trockenes DMF, 15g (49.5 mmol) **2** und 12,7g (80.1 mmol) trockenes CsF zu. Die heterogene Mischung wird 20 Stunden bei Raumtemperatur gerührt ( DC-Kontrolle Edukt **2**). Nach Zugabe von 200 ml Ethylacetat wird dreimal mit gesättigter NaCl-Lösung gewaschen. Die organische Phase wird über 150 ml Kieselgel filtriert und eingeengt. Der Rückstand kristallisiert nach Zugabe von n-Heptan/Etylacetat (6:1). Nach weiterem Umkristallisieren aus n-Heptan/Etylacetat erhält das Produkt **3a** (cis-Enantiomerengemisch). Das trans-Enantiomerengemisch **3b** wird aus der Mutterlauge nach Einengen und Chromatographie gewonnen. DC n-Heptan/Ethylacetat (1:1). R_{f} **3a** (cis) = 0.2, R_{f} **3b** (trans) = 0.3. C₁₆H₁₈FNO₃ (291.33) MS(ESI): 292 (M + H⁺).
Die Auftrennung des Enantiomerenpaares **3a** erfolgt durch chirale HPLC. Dabei wird zuerst das rechtsdrehende (+)Enantiomer (+)**3a** und danach das linksdrehende (-)Enantiomer (-)**3a** eluiert (Chiralpak AD 250 x 4.6; Acetonitril/Methanol (9:1).

Die Zuordnung der absoluten Stereochemie erfolgt durch Röngenstrukturanalyse der Camphansäureester der getrennten Diasteromeren **3.**

### cis-2-(3-(2-(4-Fluorphenyl)-oxazol-4-ylmethoxy)-cyclohexyloxy-methyl)-6-methyl-benzoesäuremethylester 5b

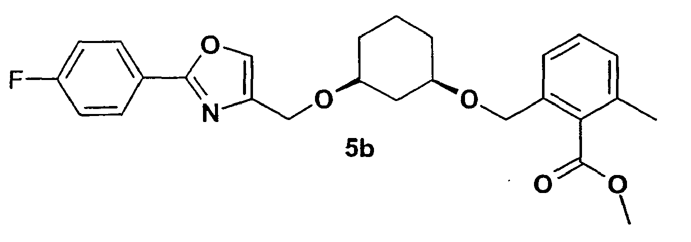

1.05 g (3.6 mmol) (-)**3a,** 1.3 g (5.4 mmol) 4 und 130 mg Kl werden in 12 ml trockenem DMF gelöst. Nach Zugabe von 140 mg (5.7 mmol) 95 %-igem NaH läßt man 1 Stunde bei Raumtemperatur rühren. Um bessere Ausbeuten bezüglich Edukt (-)**3a** zu erhalten, werden weitere 2 mal die gleiche Menge an **4** und NaH zugegeben und jeweils 1 Stunde gerührt. Dann läßt man über Nacht stehen. Die Reaktionslösung wird mit 150 ml Ethylacetat verdünnt und auf 50 ml Wasser gegossen. Nach weiterem 2 maligen Waschen mit NaCl-Lösung wird die organische Phase über Kieselgel filtriert, eingeengt und der Rückstand mit Flashchromatgraphie (n-Heptan/Ethylacetat, 1:1) gereinigt. Man erhält **5b** als farblosen, amorphen Feststoff. DC n-Heptan/Ethylacetat (1:1). R_{f} = 0.5. C₂₆H₂₈FNO₅ (453.52) MS(ESI): 454 (M + H⁺).

### (+)-cis-2-(3-(2-(4-Fluorphenyl)-oxazol-4-ylmethoxy)-cyclohexyloxy-methyl)-6-methylbenzoesäure 6b

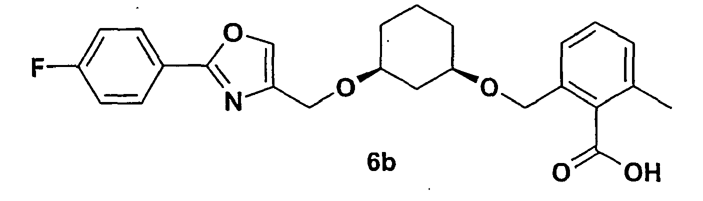

4.2 g (9.2 mmol) **5b** werden in 120 ml t-BuOH gelöst. Nach Zugabe von 50 ml 50% KOH aq. wird 24 Stunden bei 100°C gekocht. Zum Aufarbeiten läßt man abkühlen und verdünnt dann mit 100 ml Ethylacetat. Durch Zugabe von 2 N wäßriger HCl wird die wäßrige Phase leicht sauer eingestellt und weitere 2 mal mit 100 ml Ethylacetat extrahiert. Die organische Phase trocknet man über MgSO₄, filtriert, engt ein und reinigt den Rückstand mit Flashchromatographie (Methylenchlorid/Methanol/conz.Ammoniak, 30/5/1). Man erhält **6b** als weißen, amorphen Feststoff. DC (Methylenchlorid/Methanol/conz.Ammoniak, 30/5/1). R_{f} = 0.3. Umkristallisation aus Toluol. C₂₅H₂₆FNO₅ (432.42) MS(ESI): 433 (M + H⁺).

### Beispiel III

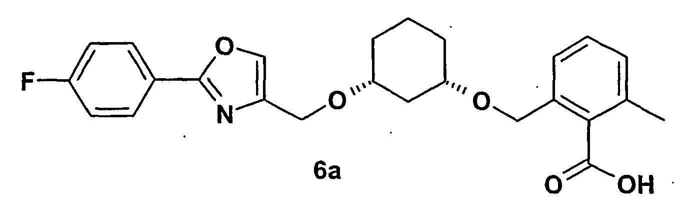

### (-)-cis-2-{3-[2-(4-Fluoro-phenyl)-oxazol-4-ylmethoxy]-cyclohexyloxymethyl}-6-methylbenzoesäure 6a

Aus (+)**3a** und 2-Brommethyl-6-methyl-benzoesäuremethylester **4** erhält man analog zu Beispiel I das Produkt **6a** mit dem Molekulargewicht 432.42 (C₂₅H₂₆FNO₅);
MS(ESI): 433 (M+H⁺).

### Beispiel IV

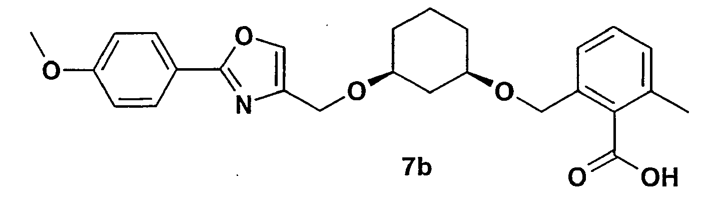

### cis-2-(3-(2-(4-Methoxyphenyl)-oxazol-4-ylmethoxy)-cyclohexyloxy-methyl)-6-methylbenzoesäure 7b

170 mg (0.39 mmol) **6b** werden in 4 ml 5.6 M NaOMe/MeOH-Lösung 20 Stunden bei 120 °C Ölbadtemperatur erhitzt. Nach Zugabe von Ethylacetat und 2 N HCl wird analog der Synthese von **6b** aufgearbeitet. Man erhält **7b** als farblosen, amorphen Feststoff. DC: (Methylenchlorid/Methanol/conz.Ammoniak, 30/5/1). R_{f} ~ 0.3.
C₂₆H₂₉NO₆ (451.52) MS(ESI): 452 (M + H⁺).
Auf gleichem Weg erhält man aus **6a** das stereoisomere 7a: 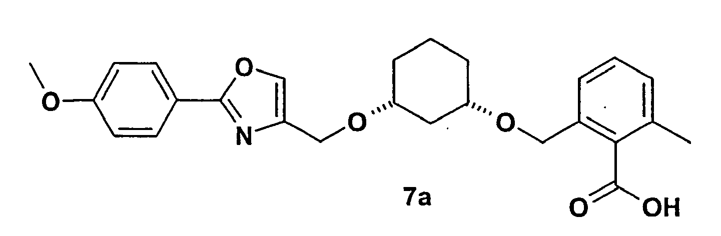 DC: (Methylenchlorid/Methanol/conz.Ammoniak, 30/5/1). R_{f} ~ 0.3.
C₂₆H₂₉NO₆ (451.52) MS(ESI): 452 (M + H⁺).

### Beispiel V (11a) und Beispiel VI (11b)

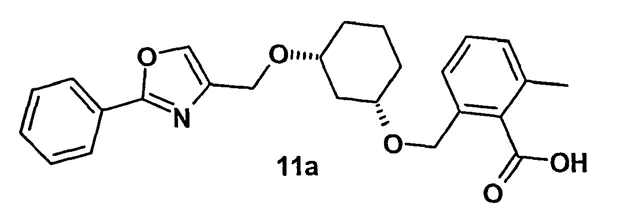

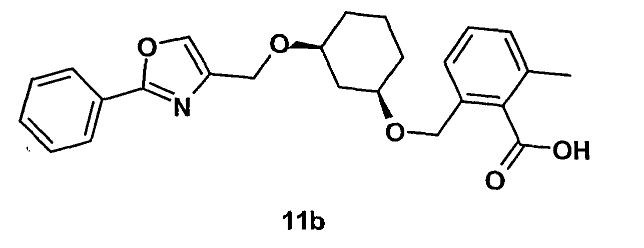

### cis-3-(2-Phenyl-oxazol-4-ylmethoxy)-cyclohexanol 12a,b

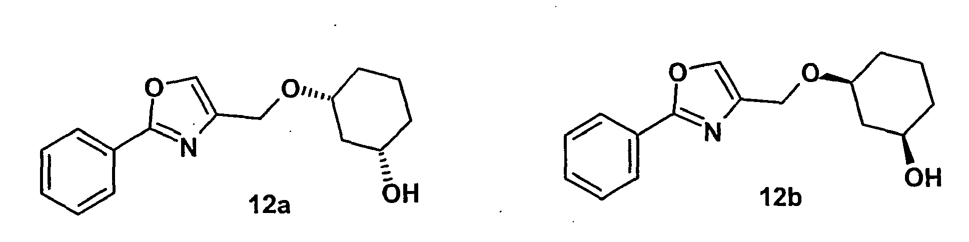

Aus 1,3-Cyclohexandiol und 4-Iodomethyl-2-phenyl-oxazol erhält man das Racemat **12** mit dem Molekulargewicht von 273.33 (C₁₆H₁₉NO₃); MS(ESI): 274 (M+H⁺).

Die Trennung der Enantiomeren erfolgt durch HPLC an einer chiralen Säule. Dabei wird zuerst das (+)-Enantiomer **12a** und danach das (-)-Enantiomer **12b** eluiert (Chiralpak OD 250x4.6; n-Heptan:Ethanol:Acetonitril = 110: 2:1 + 0.05% Trifluoressigsäure).

### cis-2-Methyl-6-[3-(2-phenyl-oxazol-4-ylmethoxy)-cyclohexyloxymethyl]-benzoesäuremethylester 13a

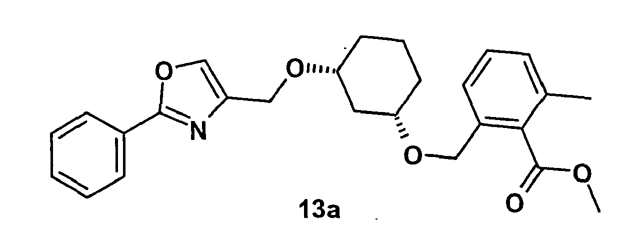

Aus **12a** und 2-Brommethyl-6-methyl-benzoesäuremethylester erhält man **13a** mit dem Molekulargewicht von 435.52 (C₂₆H₂₉NO₅); MS(ESI): 436 (M+H⁺).

### cis-2-Methyl-6-[3-(2-phenyl-oxazol-4-ylmethoxy)-cyclohexyloxymethyl]-benzoesäuremethylester 13b

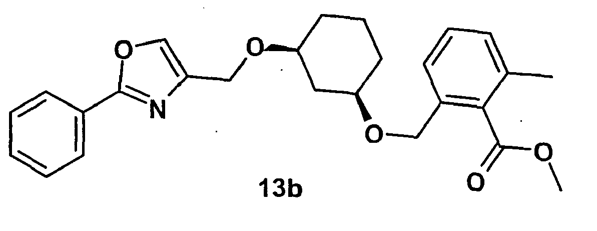

Aus **12b** und 2-Brommethyl-6-methyl-benzoesäuremethylester erhält man **13b** mit dem Molekulargewicht von 435.52 (C₂₆H₂₉NO₅); MS(ESI): 436 (M+H⁺).

### cis-2-Methyl-6-[3-(2-phenyl-oxazol-4-ylmethoxy)-cyclohexyloxymethyl]-benzoesäure 11a

Aus 13a erhält man durch Verseifung **11a** mit dem Molekulargewicht von 421.50 (C₂₅H₂₇NO₅); MS(ESI): 422 (M+H⁺).

### cis-2-Methyl-6-[3-(2-phenyl-oxazol-4-ylmethoxy)-cyclohexyloxymethyl]-benzoesäure 11b

Aus **13b** erhält man analog **11b** mit dem Molekulargewicht von 421.50 (C₂₅H₂₇NO₅); MS(ESI): 422 (M+H⁺).

### Beispiel VII (14a) und Beispiel VIII (14b)

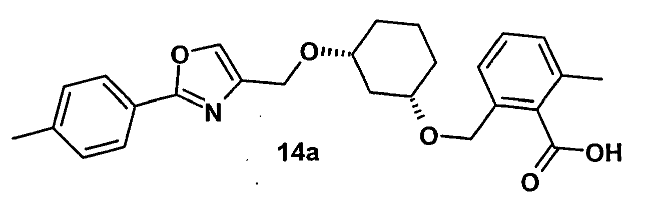

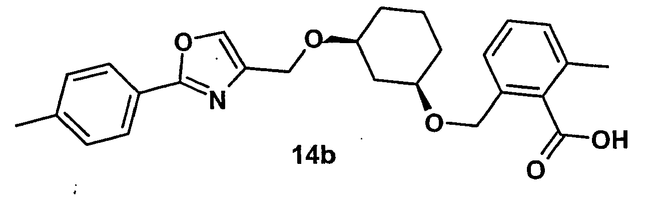

### Cis-3-(2-p-Tolyl-oxazol-4-ylmethoxy)-cyclohexanol 15a,b

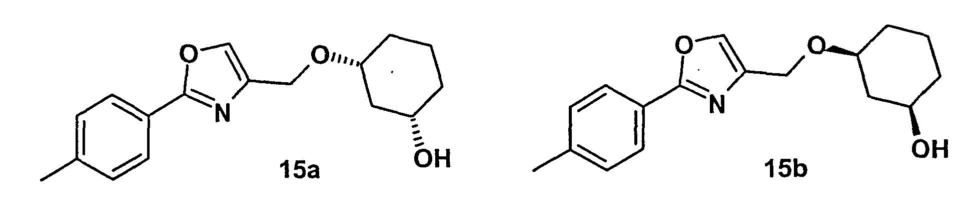

Aus Cyclohexandiol und 4-lodomethyl-2-p-tolyl-oxazol erhält man das Racemat **15** mit dem Molekulargewicht von 287.36 (C₁₇H₂₁NO₃); MS(ESI): 288 (M+H⁺).

Die Trennung der Enantiomeren erfolgt durch HPLC an einer chiralen Säule. Dabei wird zuerst das (+)-Enantiomer **15a** und danach das (-)-Enantiomer **15b** eluiert (Chiralpak OD 250x4.6; n-Heptan:Ethanol:Acetonitril = 110:5:1 + 0.05% Trifluoressigsäure).

### cis-2-Methyl-6-[3-(2-p-tolyl-oxazol-4-ylmethoxy)-cyclohexyloxymethyl]-benzoesäuremethylester 16a

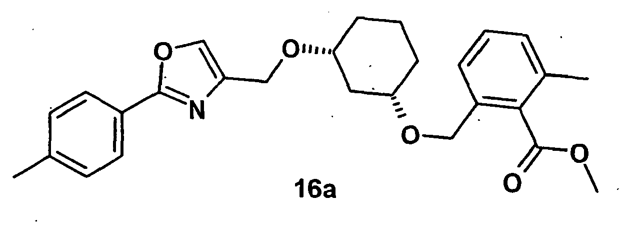

Aus **15a** und 2-Brommethyl-6-methyl-benzoesäuremethylester erhält man **16a** mit dem Molekulargewicht von 449.55 (C₂₇H₃₁NO₅); MS(ESI): 450 (M+H⁺).

### cis-2-Methyl-6-[3-(2-p-tolyl-oxazol-4-ylmethoxy)-cyclohexyloxymethyl]-benzoesäuremethylester 16b

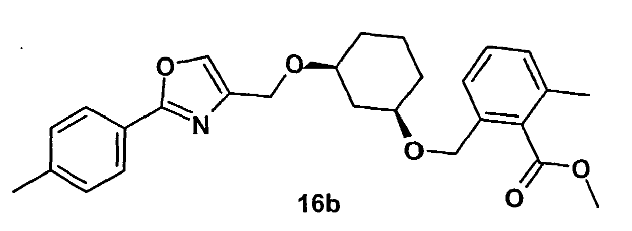

Aus **15b** und 2-Brommethyl-6-methyl-benzoesäuremethylester erhält man **16b** mit dem Molekulargewicht von 449.55 (C₂₇H₃₁NO₅); MS(ESI): 450 (M+H⁺).

### cis-2-Methyl-6-[3-(2-p-tolyl-oxazol-4-ylmethoxy)-cyclohexyloxymethyl]-benzoesäure 14a

Aus 16a erhält man **14a** mit dem Molekulargewicht von 435.52 (C₂₆H₂₉NO₅); MS(ESI): 436 (M+H⁺).

### cis-2-Methyl-6-[3-(2-p-tolyl-oxazol-4-ylmethoxy)-cyclohexyloxymethyl]-benzoesäure 14b

Aus **16b** erhält man das gewünschte Produkt mit dem Molekulargewicht von 435.52 (C₂₆H₂₉NO₅); MS(ESI): 436 (M+H⁺).

### Beispiel IX (17a) und Beispiel X (17b)

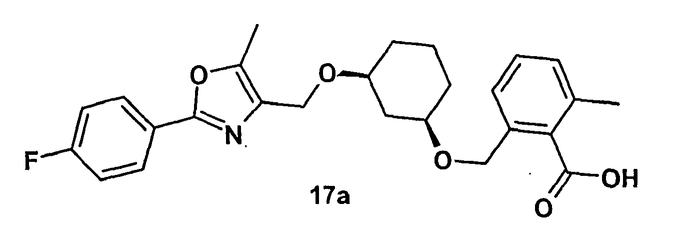

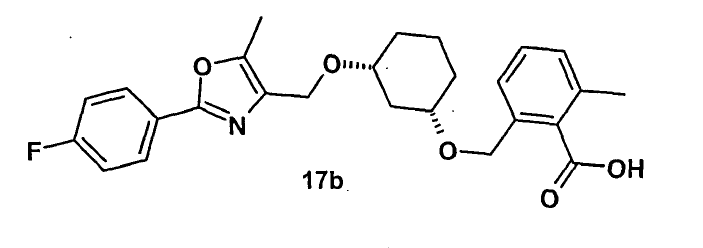

### cis-3-[2-(4-Fluoro-phenyl)-5-methyl-oxazol-4-ylmethoxy]-cyclohexanol 18a,b

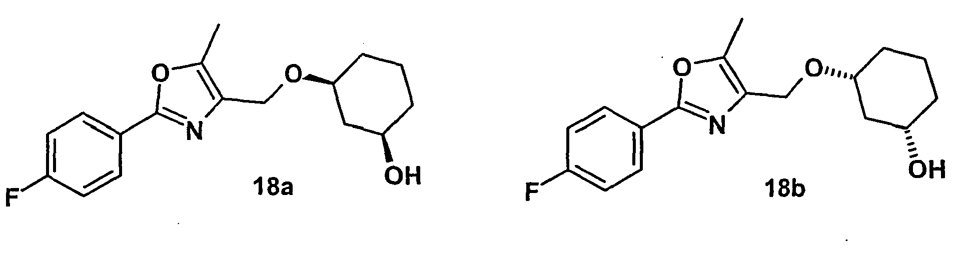

Aus Cyclohexandiol und 2-(4- Fluorphenyl)-4-iodmethyl-5-methyl-oxazol erhält man das Racemat **18** mit dem Molekulargewicht von 305.35 (C₁₇H₂₀FNO₃); MS(ESI): 306 (M+H+).

Die Trennung der Enantiomeren erfolgt durch HPLC an einer chiralen Säule. Dabei wird zuerst das (+)-Enantiomer **18a** und danach das (-)-Enantiomer **18b** eluiert (Chiralpak OD 250x4.6; n-Heptan:Ethanol:Acetonitril = 110:2:1 + 0.05% Trifluoressigsäure).

### cis-2-{3-[2-(4-Fluorphenyl)-5-methyl-oxazol-4-ylmethoxy]-cyclohexyloxymethyl}-6-methyl-benzoesäre methylester 19a

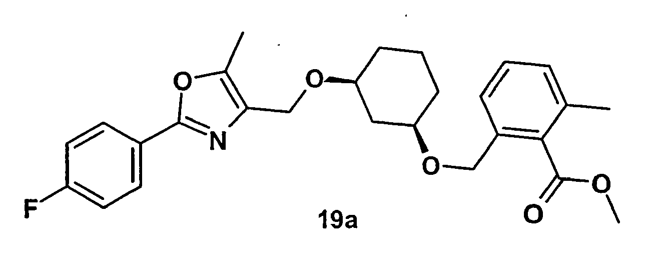

Aus **18a** und 2-Brommethyl-6-methyl-benzoesäuremethylester erhält man **19a** mit dem Molekulargewicht von 467.54 (C₂₇H₃₀FNO₅); MS(ESI): 468 (M+H⁺).

### cis-2-{3-[2-(4-Fluorphenyl)-5-methyl-oxazol-4-ylmethoxy]-cyclohexyloxymethyl}-6-methyl-benzoesäuremethylester 19b

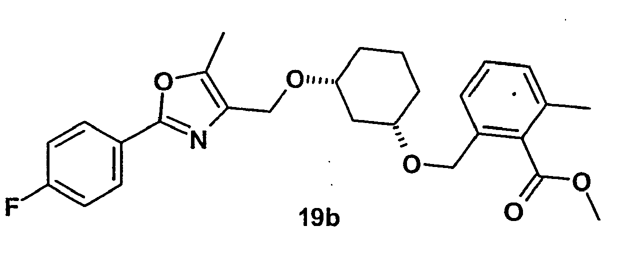

Aus **18b** und 2-Brommethyl-6-methyl-benzoesäuremethylester erhält man **19b** mit dem Molekulargewicht von 467.54 (C₂₇H₃₀FNO₅); MS(ESI): 468 (M+H⁺).

### cis-2-(3-[2-(4-fluoro-phenyl)-5-methyl-oxazol-4-ylmethoxy]-cyclohexyloxymethyl}-6-methyl- benzoesäure 17a

Aus **19a** erhält man durch Verseifung **17a** mit dem Molekulargewicht von 453.52 (C₂₆H₂₈FNO₅); MS(ESI): 454 (M+H⁺).

### cis-2-(3-[2-(4-fluoro-phenyl)-5-methyl-oxazol-4-ylmethoxy]-cyclohexyloxymethyl}-6-methyl- benzoesäure 17b

Aus **19b** erhält man analog **17b** mit dem Molekulargewicht von 453.52 (C₂₆H₂₈FNO₅); MS(ESI): 454 (M+H⁺).

### Beispiel XI (20) und Beispiel XII (21)

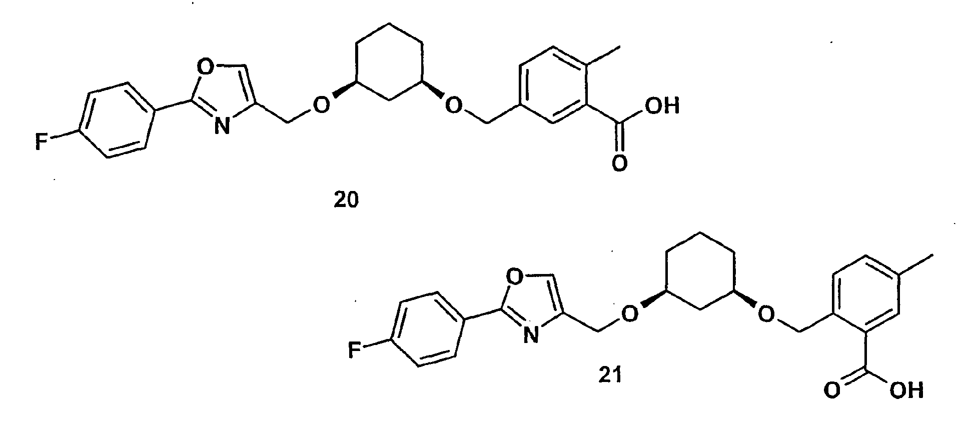

### 5-Bromomethyl-2-methyl-benzoesäureethylester 22 und 2-Bromomethyl-5-methyl-benzoesäureethylester 23

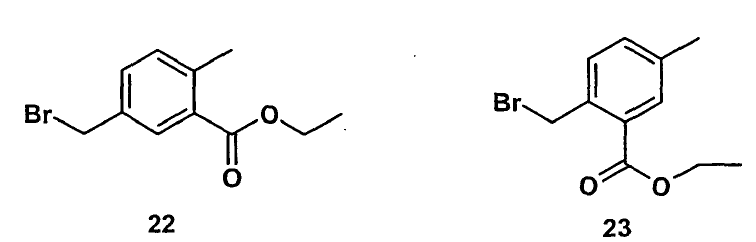

Eine Lösung von 3,5 g 2,5-Dimethyl-benzoesäureethylester, 3,15 g N-Bromsuccinimid und 100 ml Tetrachlorkohlenstoff wird für 3 Stunden zum Rückfluss unter Bestrahlung mit einer 300 Watt Photolampe erhitzt. Der entstehende Niederschlag wird abfiltriert und das eingeengte Filtrat an Kieselgel chromatographiert. Man erhält so ein angenähertes 2:3 (**22:23**) Gemisch der regioisomeren Benzylbromide **22** und **23** mit dem Molekulargewicht 257,13 (C₁₁H₁₃BrO₂); MS (ESI+): 257 (M+H⁺).

Rac-cis-5-{3-[2-(4-Fluor-phenyl)-oxazol-4-ylmethoxy]-cyclohexyloxymethyl}-2-methylbenzoesäureethylester **24** und rac-cis-2-{3-[2-(4-Fluoro-phenyl)-oxazol-4-ylmethoxy]-cyclohexyloxymethyl}-5-methyl-benzoesäureethylester **25**

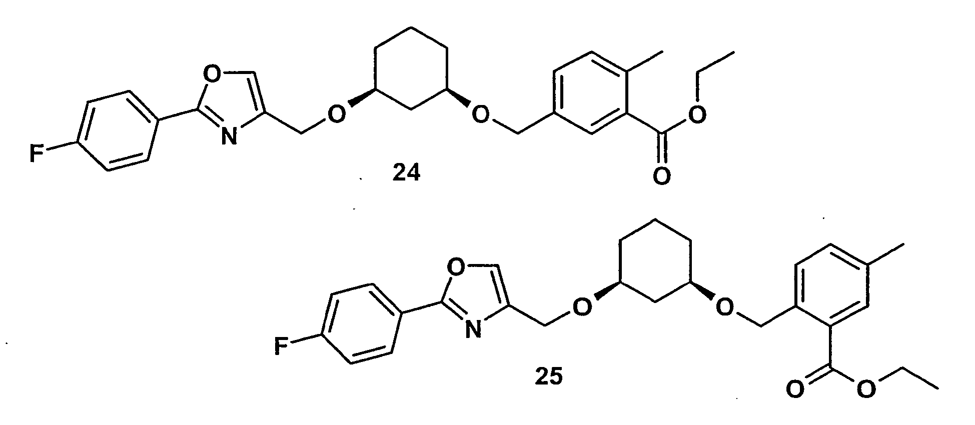

Zu einer Suspension von 40 mg Natriumhydrid (55-65% in Paraffinöl) in 1 ml Dimethylformamid bei 0 °C wird eine Lösung von 150 mg rac-cis-3-[2-(4-Fluor-phenyl)-oxazol-4-ylmethoxy]-cyclohexanol **3a** in 0.5 ml Dimethylformamid getropft. Nach beendeter Gasentwicklung werden 198 mg eines 2:3 Gemisches aus 5-Brommethyl-2-methyl-benzoesäureethylester **22** und 2-Bromomethyl-5-methyl-benzoesäureethylester **23** zugesetzt. Nach 30 Minuten bei 0 °C lässt man noch 1 Stunde bei Raumtemperatur reagieren. Es wird in Ammoniumchloridlösung gegossen und zweimal mit MTBE extrahiert. Nach dem Trocknen über Magnesiumsulfat, Filtration und Einengen am Rotationsverdampfer wird das Produkt durch Chromatographie an Kieselgel gereinigt (Eluent: n-Heptan/Ethylacetat 3:1). Man erhält so das schneller eluierende Produkt rac-cis-2-{3-[2-(4-Fluoro-phenyl)-oxazol-4-ylmethoxy]-cyclohexyloxymethyl}-5-methyl-benzoesäureethylester **25** mit dem Molekulargewicht 467,54 (C₂₇H₃₀FNO₅); MS (ESI+): 468 (M+H⁺).

Weiterhin isoliert wird das später eluierende Produkt rac-cis-5-{3-[2-(4-Fluor-phenyl)-oxazol-4-ylmethoxy]-cyclohexyloxymethyl}-2-methyl-benzoesäureethylester **24** mit dem Molekulargewicht 467,54 (C₂₇H₃₀FNO₅); MS (ESI+): 468 (M+H⁺).

### Rac-cis-5-{3-[2-(4-Fluorphenyl)-oxazol-4-ylmethoxy]-cyclohexyloxymethyl}-2-methyl-benzoesäure 20

Eine Suspension von 47 mg rac-cis-5-{3-[2-(4-Fluorphenyl)-oxazol-4-ylmethoxy]-cyclohexyloxymethyl}-2-methyl-benzoesäureethylester **24,** 2 ml 1,1-Dimethylethanol und 50 %(w/w) Kaliumhydroxid wird für 2 Stunden auf 85 °C (Ölbad) erhitzt. Es wird mit verdünnter Salzsäure auf pH 3 eingestellt und mit MTBE zweimal extrahiert. Nach dem Trocknen über Magnesiumsulfat, Filtration und Einengen am Rotationsverdampfer wird das Produkt durch Chromatographie gereinigt. Man erhält so das Produkt **20** mit dem Molekulargewicht 439,49 (C₂₅H₂₆FNO₅); MS (ESI+): 440 (M+H⁺).

Analog zu **20** wird hergestellt:
Rac-cis-2-{3-[2-(4-Fluorphenyl)-oxazol-4-ylmethoxy]-cyclohexyloxymethyl}-5-methylbenzoesäure **21**
aus rac-cis-2-{3-[2-(4-Fluorphenyl)-oxazol-4-ylmethoxy]-cyclohexyloxymethyl}-5-methyl-benzoesäureethylester **25.**

### Beispiel XIII

### rac-trans-2-{3-[2-(4-Fluor-phenyl)-oxazol-4-ylmethoxy]-cyclohexyloxymethyl}-6-methylbenzoesäure 26

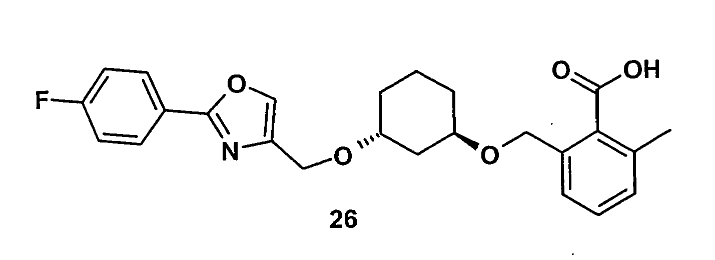

Aus rac-trans **3b** und 2-Brommethyl-6-methyl-benzoesäuremethylester erhält man das Produkt **26** mit dem Molekulargewicht von 439.49 (C₂₅H₂₆FNO₅); MS(ESI): 440 (M+H⁺).

### Beispiel XIV

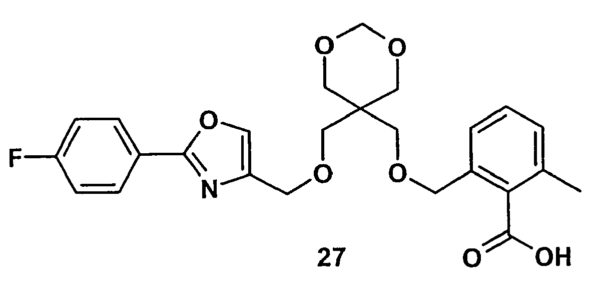

### 5-(2-(4-Fluorphenyl)-oxazol-4ylmethoxymethyl)-1,3-dioxan-5yl-methanol 28

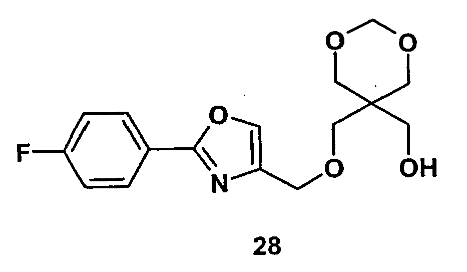

1.0 g (6.7 mmol) 5-hydroxymethyl-(1,3)dioxan-5-yl-methanol und 0.5 g (16.5 mmol) **2** werden in 20 ml trockenem DMF gelöst. Nach Zugabe von 300 mg 55%-igem NaH in Paraffinöl läßt man 1 Stunde bei Raumtemperatur rühren. Die Aufarbeitung erfolgt analog der Synthese von Verbindung **5b**. Man erhält **28** als weißen amorphen Feststoff. DC (n-Heptan/Ethylacetat 1:2). R_{f} = 0.4. C₁₆H₁₈FNO₅ (323.33) MS 324.2 M + H⁺.

### 2-{5-[2-(4-Fluorphenyl)-oxazol-4-ylmethoxymethyl]-[1,3]dioxan-5-ylmethoxymethyl}-6-methyl-benzoesäure methylester 29

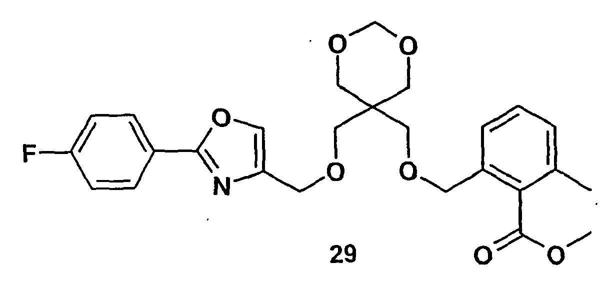

Verbindung **29** wird analog der Synthese von **5b** aus **28** und **4** und hergestellt.

### 2-{5-[2-(4-Fluorphenyl)-oxazol-4-ylmethoxymethyl]-[1,3]dioxan-5-ylmethoxymethyl}-6-methyl-benzoesäure 27

Verbindung **27** wird analog der Synthese von **6b** aus **29** durch Verseifung hergestellt.

### Beispiel XV

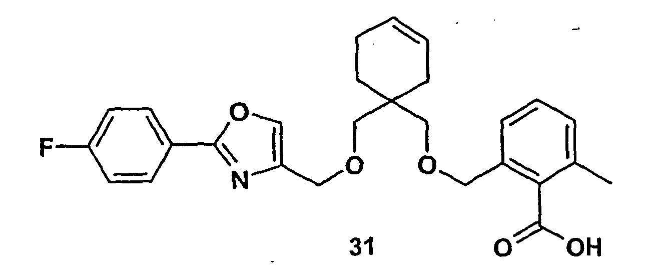

### 2-{1-[2-(4-Fluorophenyl)-oxazol-4-ylmethoxymethyl]-cyclohex-3-enylmethoxymethyl}-6-methyl-benzoesäure 31

Ausgehend von (1-Hydroxymethyl-cyclohex-3-enyl)-methanol, Iodid **2** und Bromid **4** erhält man wie für **27** beschrieben das Produkt **31** mit dem Molekulargewicht von 465.53 (C₂₇H₂₈FNO₅); MS(ESI): 466 (M+H⁺).

### Beispiel XVI

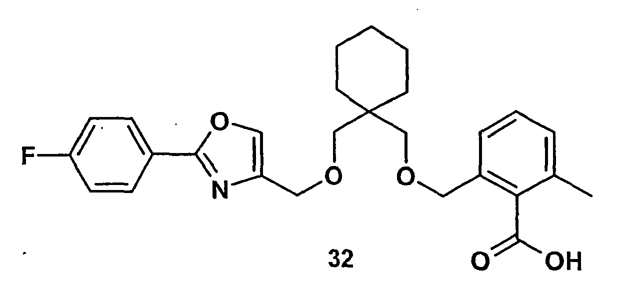

### 2-{1-[2-(4-Fluorphenyl)-oxazol-4-ylmethoxymethyl]-cyclohexylmethoxymethyl}-6-methyl-benzoesäure 32

Ausgehend von (1-Hydroxymethyl-cyclohexyl)-methanol, Iodid **2** und Bromid **4** erhält man auf analogem Weg wie für 27 beschrieben das Produkt **32** mit dem Molekulargewicht von 467.53 (C₂₇H₃₀FNO₅); MS(ESI): 468 (M+H⁺).

### Beispiel XVII

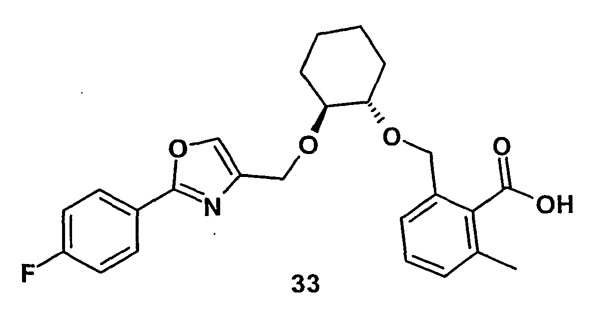

### rac-trans-2-{2-[2-(4-Fluorphenyl)-oxazol-4-ylmethoxy]-cyclohexyloxymethyl}-6-methylbenzoesäure 33

Aus trans-1,2-Dihydroxy-cyclohexanol, Iodid **2** und Bromid **4** erhält man analog zu **27** das gewünschte Produkt mit dem Molekulargewicht von 439.49 (C₂₅H₂₆FNO₅); MS(ESI): 440 (M+H⁺).

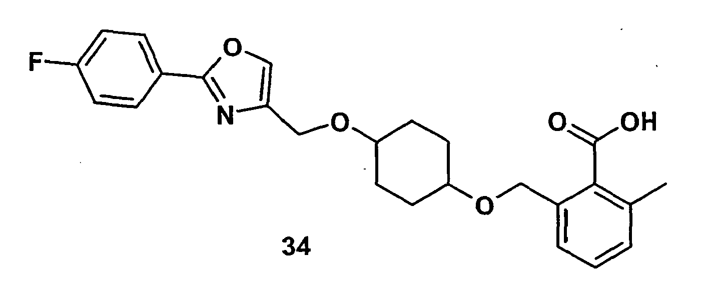

### Beispiel XVIII

### 2-{4-[2-(4-Fluorphenyl)-oxazol-4-ylmethoxy]-cyclohexyloxymethyl}-6-methylbenzoesäure 34

Aus 1,4-Cyclohexandiol, Iodid **2** und Bromid **4** erhält man **34** mit dem Molekulargewicht von 439.49 (C₂₅H₂₆FNO₅); MS(ESI): 440 (M+H⁺).

### Beispiel XIX

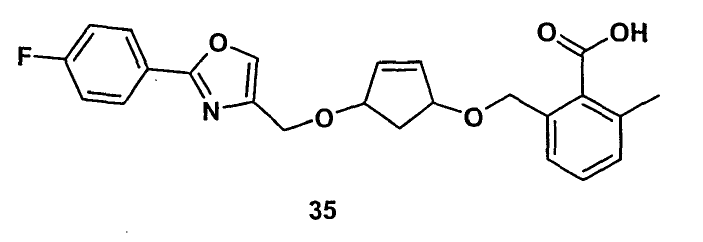

### 2-{4-[2-(4-Fluorphenyl)-oxazol-4-ylmethoxy]-cyclopent-2-enyloxymethyl}-6-methylbenzoesäure 35

Aus Cyclopent-2-en-1,4-diol, Iodid **2** und Bromid **4** erhält man das Produkt **35** mit dem Molekulargewicht von 423.45 (C₂₄H₂₂FNO₅); MS(ESI): 424 (M+H⁺).

### Beispiel XX

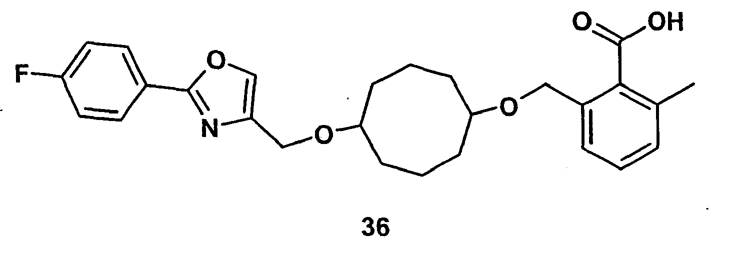

### 2-{5-[2-(4-Fluorphenyl)-oxazol-4-yl]-methoxy}-cyclooctyloxymethyl}-6-methylbenzoesäure 36

Aus 1,5-Cycloctandiol, Iodid **2** und Bromid **4** erhält man **36** mit dem Molekulargewicht von 467.54 (C₂₇H₃₀FNO₅); MS(ESI): 468 (M+H⁺).

### Beispiel XXI

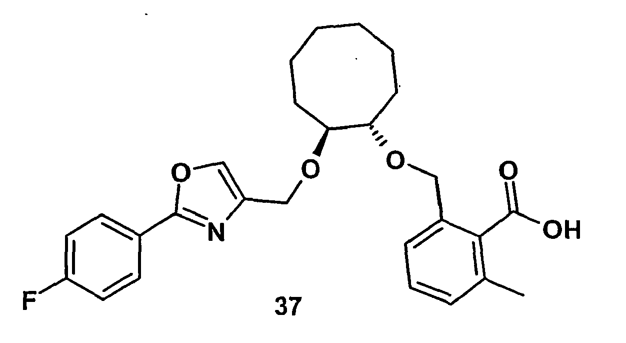

### rac-trans-2-{2-[2-(4-Fluoro-phenyl)-oxazol-4-ylmethoxy]-cyclooctyloxymethyl}-6-methylbenzoesäure 37

Aus trans-1,2-Cycloctandiol, Iodid **2** und Bromid **4** erhält man das gewünschte Produkt mit dem Molekulargewicht von 467.54 (C₂₇H₃₀FNO₅); MS(ESI): 468 (M+H⁺).

### Beispiel XXII

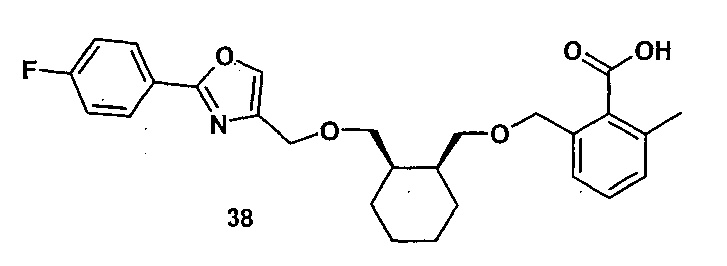

### rac-cis-2-{2-[2-(4-Fluorphenyl)-oxazol-4-ylmethoxy]methyl-cyclohexylmethoxymethyl}-6-methyl-benzoesäure 38

Aus cis-(2-Hydroxymethyl-cyclohexyl)-methanol, Iodid **2** und Bromid **4** erhält man das Produkt 38 mit dem Molekulargewicht von 467.54 (C₂₇H₃₀FNO₅); MS(ESI): 468 (M+H⁺).

### Beispiel XXIII

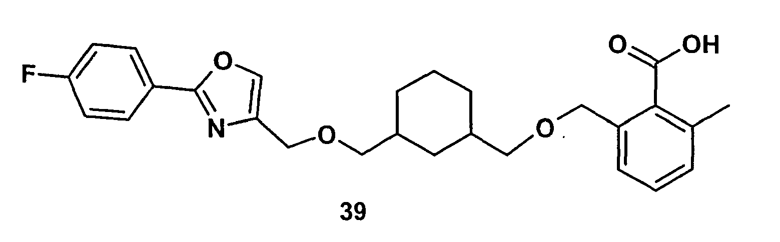

### 2-{2-[2-(4-Fluorphenyl)-oxazol-4-ylmethoxy]methyl-cyclohexylmethoxymethyl}-6-methyl-benzoesäure 39

Aus (3-Hydroxymethyl-cyclohexyl)-methanol, Iodid **2** und Bromid **4** erhält man das Produkt 39 mit dem Molekulargewicht von 467.54 (C₂₇H₃₀FNO₅); MS(ESI): 468 (M+H⁺).

### Beispiel XXIV

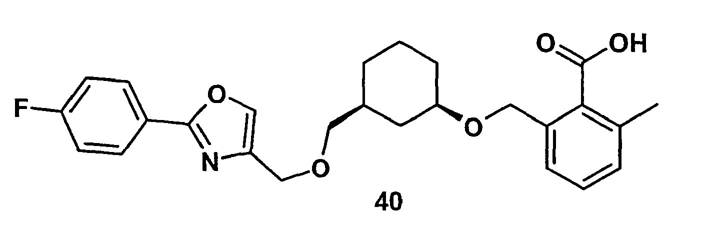

### rac-cis 2-{3-[2-(4-Fluorphenylroxazol-4-ylmethoxymethyl]-cyclohexyloxymethyl}-6-methyl-benzoesäure 40

Aus cis-3-Hydroxymethylcyclohexanol, Iodid **2** und Bromid **4** erhält man **40** mit dem Molekulargewicht von 453.52 (C₂₆H₂₈FNO₅); MS(ESI): 454 (M+H⁺).

### Beispiel XXV

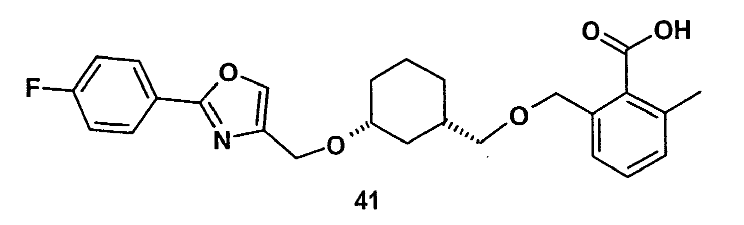

### rac-cis-2-{3-[2-(4-Fluorphenyl)-oxazol-4-ylmethoxy]-cyclohexylmethoxymethyl}-6-methyl-benzoesäure 41

Aus cis-3-Hydroxymethylcyclohexanol, Bromid **4** und Iodid **2** (Umkehrung der Reaktionsfolge) erhält man das Produkt **41** mit dem Molekulargewicht von 453.52 (C₂₆H₂₈FNO₅); MS(ESI): 454 (M+H⁺).

### Beispiel XXVI

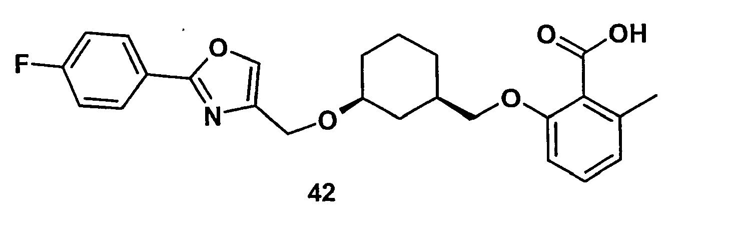

### rac-cis-2-{3-[2-(4-Fluorphenyl)-oxazol-4-ylmethoxy]-cyclohexylmethoxy}-6-methylbenzoesäure 42

Aus cis-3-Hydroxymethylcyclohexanol, Iodid **2** und 2-Hydroxy-6-methylbenzoesäureethylester erhält man das Produkt **42** mit dem Molekulargewicht von 439.49 (C₂₅H₂₆FNO₅); MS(ESI): 440 (M+H⁺).

### Beispiel XXVII

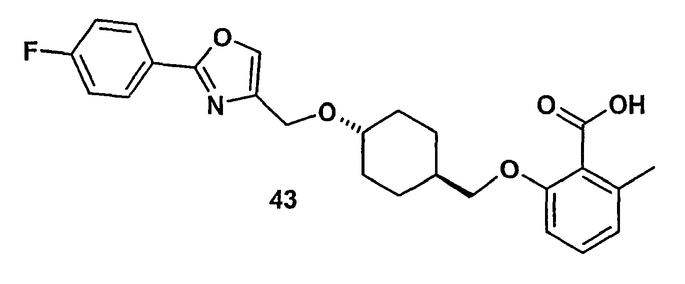

### rac-trans-2-{4-[2-(4-Fluorphenyl)-oxazol-4-ylmethoxy]-cyclohexylmethoxy}-6-methylbenzoesäure 43

Aus trans-4-Hydroxymethylcyclohexanol, Iodid **2** und 2-Hydroxy-6-methylbenzoesäureethylester erhält man das Produkt **43** mit dem Molekulargewicht von 439.49 (C₂₅H₂₆FNO₅); MS(ESI): 440 (M+H⁺).

### Beispiel XXVIII

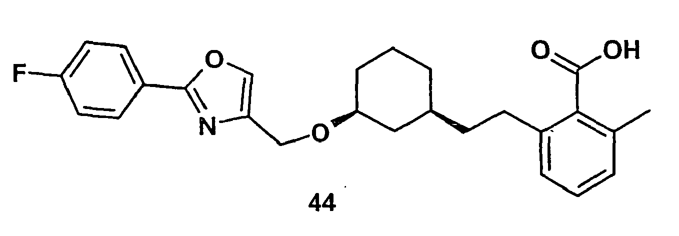

### rac-cis-2-(2-{3-[2-(4-Fluorphenyl)-oxazol-4-ylmethoxy]-cyclohexyl}-ethyl)-6-methylbenzoesäure 44

Aus cis-3-Ethinyl-cyclohex-2-enol, 2-Methyl-6-trifluormethansulfonyloxy-benzoesäureethylester und Iodid **2** erhält man das Produkt **44** mit dem Molekulargewicht von 437.52 (C₂₆H₂₈FNO₄); MS(ESI): 438 (M+H⁺).

### Beispiel XXIX

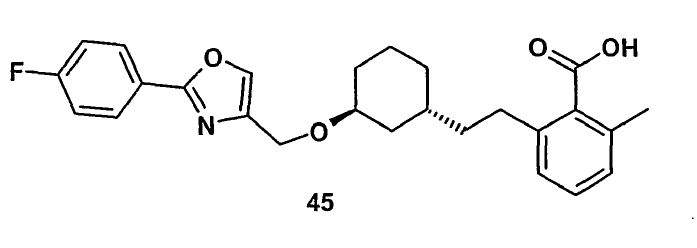

### rac-trans-2-(2-{3-[2-(4-Fluorphenyl)-oxazol-4-ylmethoxy]-cyclohexyl}-ethyl)-6-methylbenzoesäure 45

Aus trans-3-Ethinyl-cyclohex-2-enol, 2-Methyl-6-trifluormethansulfonyloxybenzoesäure-ethylester und Iodid **2** erhält man das Produkt **45** mit dem Molekulargewicht von 437.52 (C₂₆H₂₈FNO₄); MS(ESI): 438 (M+H⁺).

### Beispiel XXX

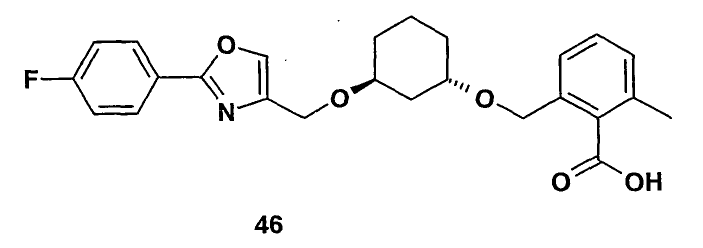

### rac-trans-2-(3-(2-(4-Fluorphenyl)-oxazol-4-ylmethoxy)-cyclohexyloxy-methyl)-6-methylbenzoesäure 46

Aus dem racemischen trans-Enantiomerengemisch **3b** (siehe Beispiel I) und 2-Brommethyl-6-methyl-benzoesäuremethylester **4** erhält man das gewünschte Produkt mit dem Molekulargewicht 439.49 (C₂₅H₂₆FNO₅); MS(ESI): 440 (M+H+).

### Beispiel XXXI

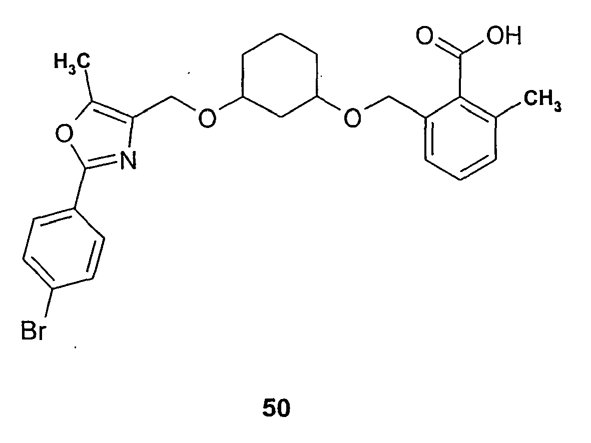

### 2-(cis-3-Hydroxy-cyclohexyloxymethyl)-6-methyl-benzoesäuremethylester 47 und 2-(trans)-3-Hydroxy-cyclohexyloxymethyl)-6-methyl-benzoesäuremethylester 48

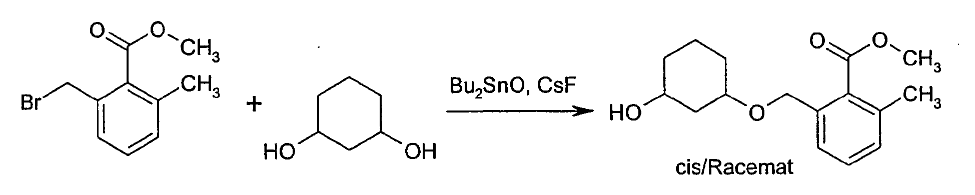

8.7 g 1,3-Cyclohexandiol werden mit 12 g Dibutylzinnoxid in 600 ml Toluol gelöst und unter Rückfluß am Wasserabscheider zum Sieden erhitzt. Das Reaktionsvolumen wird während der Reaktionsdauer auf die Hälfte reduziert. Nach 4 Std. wird das Reaktionsgemisch auf Raumtemp. gekühlt und mit 300 ml DMF, 9.0 g 2-Brommethyl-6-methyl-benzoesäuremethylester und 9.4 g Cäsiumfluorid versetzt. Man rührt 12 Std. bei Raumtemp. nach. Das Reaktionsgemisch wird durch Zugabe von Ethylacetat verdünnt und mit gesättigter NaCl-Lösung gewaschen. Die organische Phase wird über Magnesiumsulfat getrocknet, das Lösungsmittel im Vakuum entfernt und der Rückstand durch Flash-Chromatographie an Kieselgel (n-Heptan/Ethylacetat = 50:1 -> 1:2) gereinigt. Man erhält ca. 6 g des Alkohols **47** (cis-Racemat) als Öl. C₁₆H₂₂O₄ (278.35), MS(ESI): 279 (M + H⁺). Das unumgesetzte trans-1,3-Cyclohexandiol wird ebenfalls von der Chromatographiesäule eluiert. Es wird analog zu Beispiel I unter Verwendung von Natriumhydrid und 2-Brommethyl-6-methyl-benzoesäuremethylester alkyliert. Nach analoger Aufarbeitung und Chromatographie wie für das cis-Racemat beschrieben erhält man das trans-Racemat **48** C₁₆H₂₂O₄ (278.35), MS(ESI): 279 (M + H⁺).

Die Racemate **47** und **48** werden durch Chromatographie an chiraler Phase getrennt (Chiralpak AD/2 250x4,6; n-Heptan:Ethanol:Methanol = 25:1:0.5 + 0.1 % Trifluoressigsäure, Rₜ **(47a)** = 8.9 min; Retentionszeit des Enantiomers: Rₜ **(47b)** = 9.9 min (Absolute Retentionszeiten varrieren mit exakten Chromatographie-bedingungen) Die im folgenden beschriebenen Umsetzungen können sowohl mit den reinen Stereoisomeren wie auch mit Gemischen der Stereoisomere durchgeführt werden.

### 2-{3-[2-(4-Brom-phenyl)-5-methyl-oxazol-4-ylmethoxy]-cyclohexyloxy-methyl}-6-methyl- benzoesäuremethylester 49

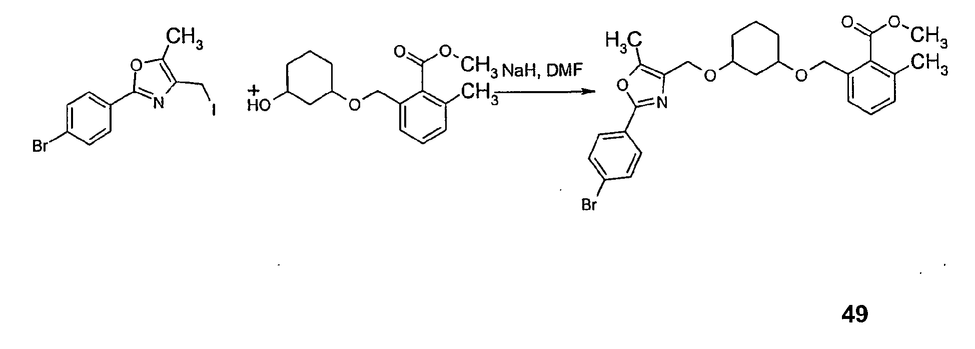

Zu einer Lösung von 200 mg 2-(3-Hydroxy-cyclohexyloxymethyl)-6-methylbenzoesäuremethylester in 5 ml Dimethylformamid werden bei Raumtemp. 50 mg 60-proz. Natriumhydrid-Suspension gegeben und anschließend 408 mg 2-(4-Bromphenyl)-4-iodmethyl-5-methyl-oxazol. Nach einer Std. wird Methyl-tert.-butylether zugegeben und mit Wasser extrahiert. Die organische Phase wird über Magnesiumsulfat getrocknet, die Lösungsmittel im Vakuum entfernt und der Rückstand durch RP-HPLC gereinigt. Man erhält **49** als hellgelbes Öl. C₂₇H₃₀BrNO₅ (528.45), MS(ESI): 528.2, 530.2 (M + H⁺).

### 2-{3-[2-(4-Brom-phenyl)-5-methyl-oxazol-4-ylmethoxy]-cyclohexyloxy-methyl}-6-methyl- benzoesäure 50

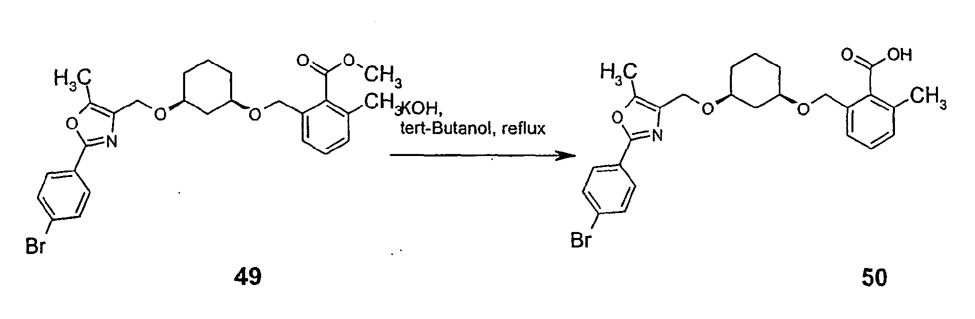

117 mg **49** werden in einer Mischung aus 10 ml tert.-Butanol und 1 ml 10 N Kaliumhydroxidlauge bei 90°C gerührt. Nach zwei Tagen wird mit Salzsäure angesäuert und mit Ethylacetat extrahiert. Die vereinigten organischen Phasen werden über Magnesiumsulfat getrocknet, die Lösungsmittel im Vakuum entfernt und der Rückstand durch RP-HPLC gereinigt. Man erhält **50** als amorphen Feststoff. C₂₆H₂₈BrNO₅ (514.52), MS(ESI): 514.29, 516.29 (M + H⁺).

### Beispiel XXXII

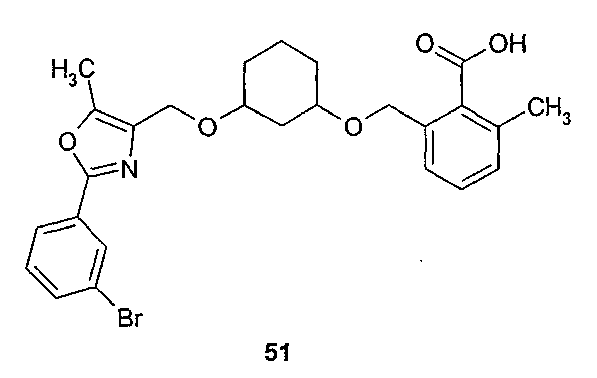

### 2-{3-[2-(3-Brom-phenyl)-5-methyl-oxazol-4-ylmethoxy]-cyclohexyloxy-methyl}-6-methyl- benzoesäure 51

Aus 2-(3-Hydroxy-cyclohexyloxymethyl)-6-methyl-benzoesäuremethylester und 2-(3-Brom-phenyl)-4-iodmethyl-5-methyl-oxazol erhält man analog zu Beispiel **50** das Produkt **51** mit dem Molekulargewicht 514.42 (C₂₆H₂₈BrNO₅), MS(ESI): 514.30,516.30 (M + H⁺).

### Beispiel XXXIII

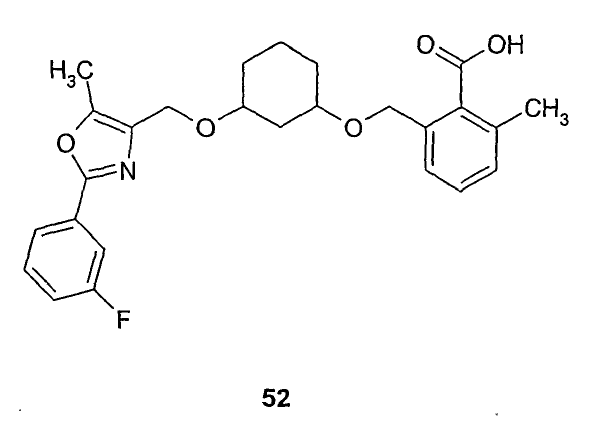

### 2-{3-[2-(3-Fluor-phenyl)-5-methyl-oxazol-4-ylmethoxy]-cyclohexyloxy-methyl}-6-methylbenzoesäure 52

Aus 2-(3-Hydroxy-cyclohexyloxymethyl)-6-methyl-benzoesäuremethylester und 2-(3-Fluor-phenyl)-4-iodmethyl-5-methyl-oxazol erhält man analog zu **50** das Produkt **52** mit dem Molekulargewicht 453.52 (C₂₆H₂₈FNO₅), MS(ESI): 454.35 (M + H⁺).

### Beispiel XXXIV

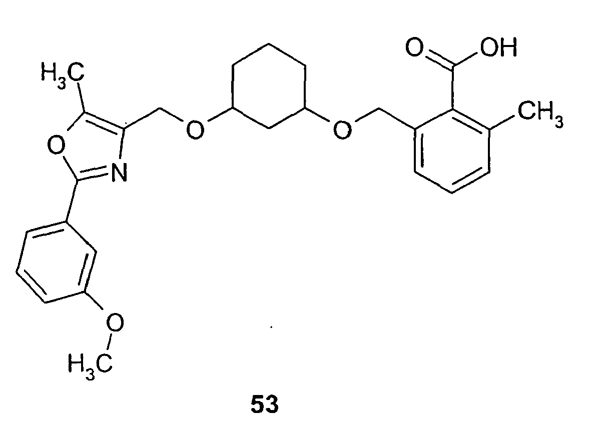

### 2-{3-[2-(3-Methoxy-phenyl)-5-methyl-oxazol-4-ylmethoxy]-cyclohexyloxy-methyl}-6-methyl- benzoesäure 53

Aus 2-(3-Hydroxy-cyclohexyloxymethyl)-6-methyl-benzoesäuremethylester und 2-(3-Methoxy-phenyl)-4-iodmethyl-5-methyl-oxazol erhält man analog zu **50** das Produkt **53** mit dem Molekulargewicht 465.55 (C₂₇H₃₁NO₆), MS(ESI): 466.37 (M + H⁺).

### Beispiel XXXV

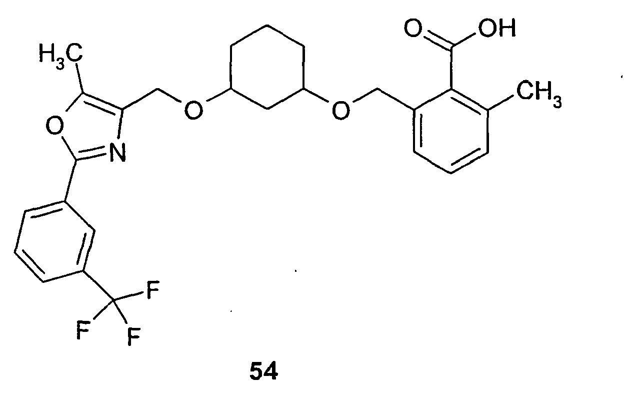

### 2-{3-[2-(3-Trifluormethyl-phenyl)-5-methyl-oxazol-4-ylmethoxy]-cyclohexyloxy-methyl}-6-methyl- benzoesäure 54

Aus 2-(3-Hydroxy-cyclohexyloxymethyl)-6-methyl-benzoesäuremethylester und 2-(3-Trifluormethyl-phenyl)-4-iodmethyl-5-methyl-oxazol erhält man analog zu **50** das Produkt **54** mit dem Molekulargewicht 503.52 (C₂₇H₂₈F₃NO₅), MS(ESI): 504.37 (M + H⁺).

### Beispiel XXXVI

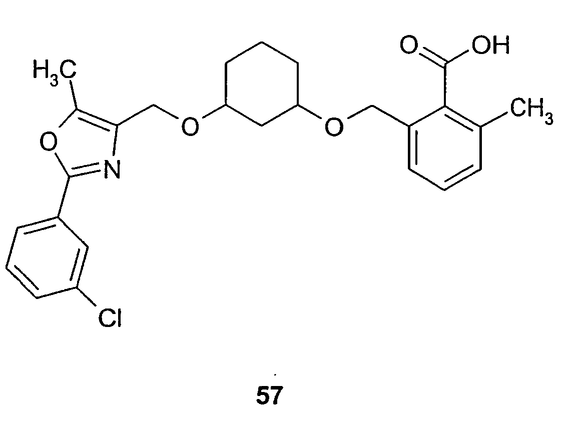

### 2-{3-[2-(3-Chlor-phenyl)-5-methyl-oxazol-4-ylmethoxy]-cyclohexyloxy-methyl}-6-methyl- benzoesäure 57

Aus 2-(3-Hydroxy-cyclohexyloxymethyl)-6-methyl-benzoesäuremethylester und 2-(3-Chlor-phenyl)-4-iodmethyl-5-methyl-oxazole erhält man analog zu **50** das Produkt **57** mit dem Molekulargewicht 469.97 (C₂₆H₂₈ClNO₅), MS(ESI): 470.43 (M + H⁺).

### Beispiel XXXVII

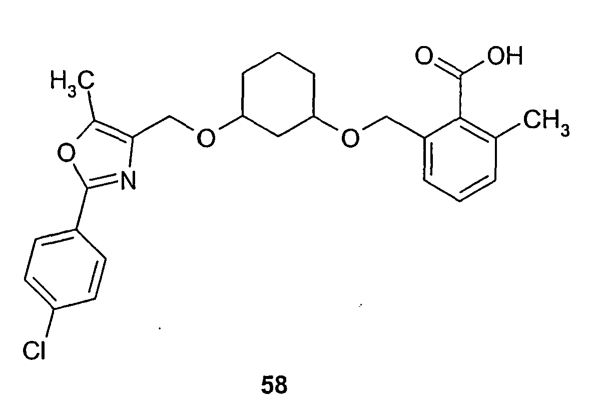

### 2-{3-[2-(4-Chlor-phenyl)-5-methyl-oxazol-4-ylmethoxy]-cyclohexyloxy-methyl}-6-methyl- benzoesäure 58

Aus 2-(3-Hydroxy-cyclohexyloxymethyl)-6-methyl-benzoesäuremethylester und 2-(4-Chlor-phenyl)-4-iodmethyl-5-methyl-oxazol erhält man analog zu **50** das Produkt **58** mit dem Molekulargewicht 469.97 (C₂₆H₂₈ClNO₅), MS(ESI): 470.40 (M + H⁺).

### Beispiel XXXVIII

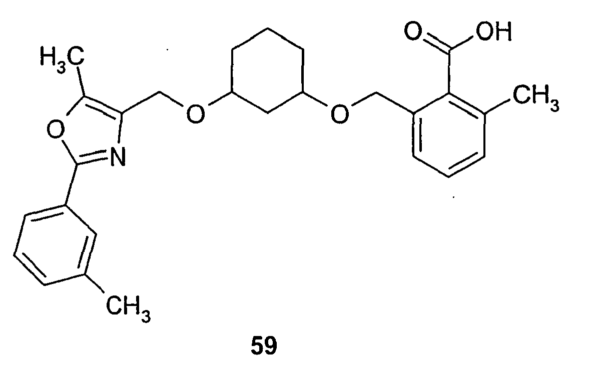

### 2-{3-[2-(3-Methyl-phenyl)-5-methyl-oxazol-4-ylmethoxy]-cyclohexyloxy-methyl}-6-methyl- benzoesäure 59

Aus 2-(3-Hydroxy-cyclohexyloxymethyl)-6-methyl-benzoesäuremethylester und 2-(3-Methyl-phenyl)-4-iodmethyl-5-methyl-oxazole erhält man analog zu **50** das Produkt **59** mit dem Molekulargewicht 449.55 (C₂₇H₃₁NO₅), MS(ESI): 450.53 (M + H⁺).

### Beispiel XXXIX

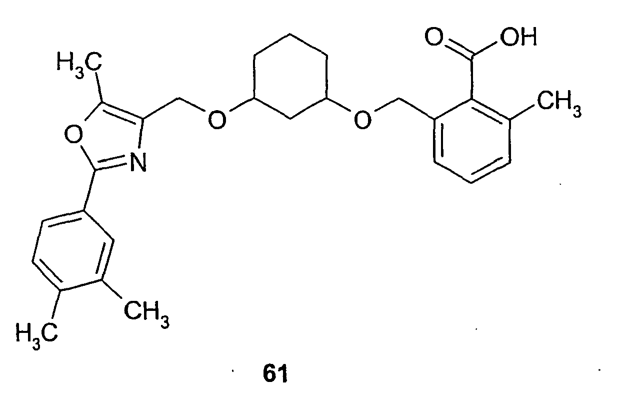

### 2-{3-[2-(3,4-Dimethyl-phenyl)-5-methyl-oxazol-4-ylmethoxy]-cyclohexyloxy-methyl}-6-methyl- benzoesäure 61

Aus 2-(3-Hydroxy-cyclohexyloxymethyl)-6-methyl-benzoesäuremethylester und 2-(3,4-Dimethyl-phenyl)-4-iodmethyl-5-methyl-oxazole erhält man analog zu **50** das Produkt **61** mit dem Molekulargewicht 463.58 (C₂₈H₃₃NO₅), MS(ESI): 464.22 (M ⁺ H⁺).

### Beispiel XL

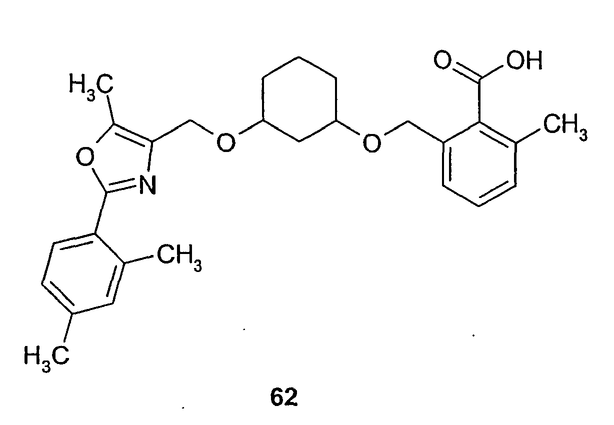

### 2-{3-[2-(2,4-Dimethyl-phenyl)-5-methyl-oxazol-4-ylmethoxy]-cyclohexyloxy-methyl}-6-methyl- benzoesäure 62

Aus 2-(3-Hydroxy-cyclohexyloxymethyl)-6-methyl-benzoesäuremethylester und 2-(2,4-Dimethyl-phenyl)-4-iodmethyl-5-methyl-oxazole erhält man analog zu **50** das Produkt **62** mit dem Molekulargewicht 463.58 (C₂₈H₃₃NO₅), MS(ESI): 464.22 (M + H⁺).

### Beispiel XLI

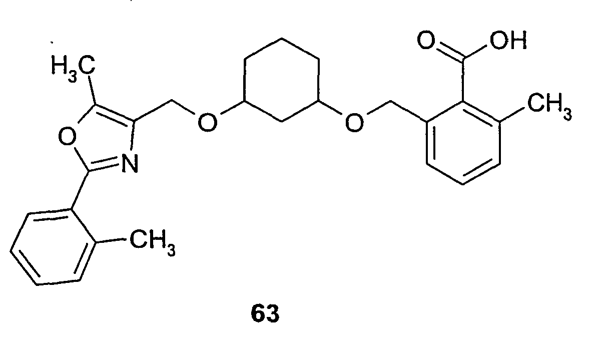

### 2-{3-[2-(2-Methyl-phenyl)-5-methyl-oxazol-4-ylmethoxy]-cyclohexyloxy-methyl}-6-methyl- benzoesäure 63

Aus 2-(3-Hydroxy-cyclohexyloxymethyl)-6-methyl-benzoesäuremethylester und 2-(2-Methyl-phenyl)-4-iodmethyl-5-methyl-oxazol erhält man analog zu **50** das Produkt **63** mit dem Molekulargewicht 449.55 (C₂₇H₃₁NO₅), MS(ESI): 450.20 (M + H⁺).

### Beispiel XLII

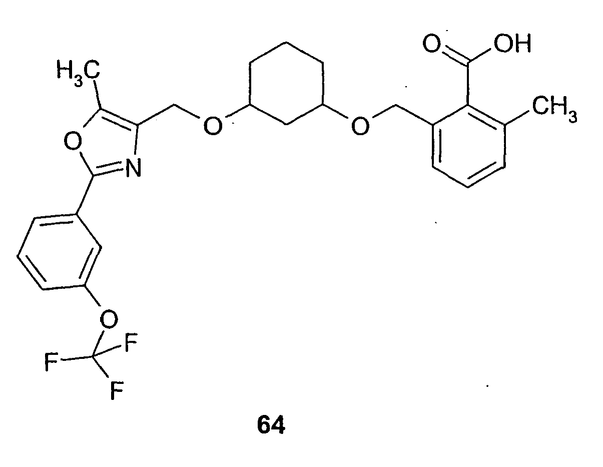

### 2-{3-[2-(3-Trifluormethoxy-phenyl)-5-methyl-oxazol-4-ylmethoxy]-cyclohexyloxymethyl}-6-methyl- benzoesäure 64

Aus 2-(3-Hydroxy-cyclohexyloxymethyl)-6-methyl-benzoesäuremethylester und 2-(3- Trifluormethoxy -phenyl)-4-iodmethyl-5-methyl-oxazol erhält man analog zu **50** das Produkt **64** mit dem Molekulargewicht 519.52 (C₂₇H₂₈F₃NO₆), MS(ESI): 520.20 (M + H⁺.

### Beispiel XLIII

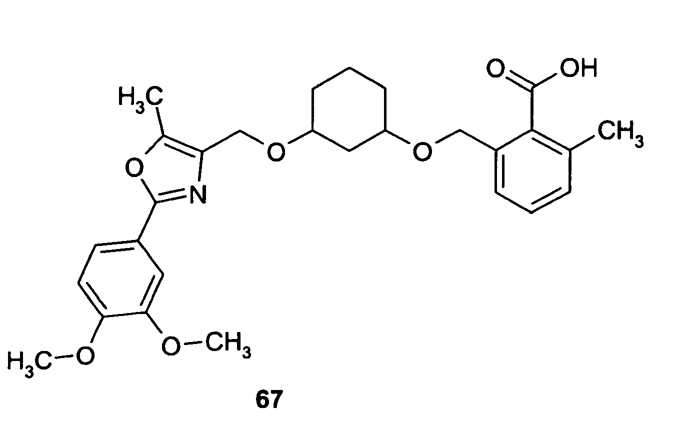

### 2-{3-[2-(3,4-Dimethoxy-phenyl)-5-methyl-oxazol-4-ylmethoxy]-cyclohexyloxy-methyl}-6-methyl- benzoesäure 67

Aus 2-(3-Hydroxy-cyclohexyloxymethyl)-6-methyl-benzoesäuremethylester und 2-(3,4- Dimethoxy -phenyl)-4-iodmethyl-5-methyl-oxazol erhält man analog zu **50** das Produkt **67** mit dem Molekulargewicht 495.58 (C₂₈H₃₃NO₇), MS(ESI): 496.20 (M + H⁺).

### Vergleichs-Beispiel XLIV

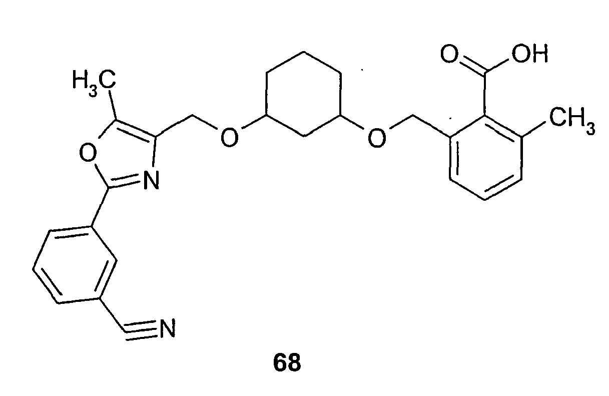

### 2-{3-[2-(3-Cyano-phenyl)-5-methyl-oxazol-4-ylmethoxy]-cyclohexyloxy-methyl}-6-methyl- benzoesäure 68

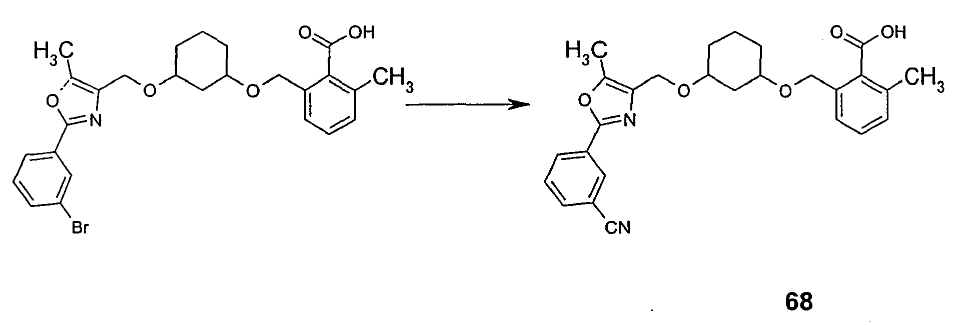

13 mg 2-{3-[2-(3-Bromo-phenyl)-5-methyl-oxazol-4-ylmethoxy]-cyclohexyloxy -methyl}-6-methyl- benzoesäure und 25 mg Zinkcyanid werden in 5 ml Dimethylformamid gelöst. Das Reaktionsgemisch wird entgast, mit Argon beschickt und mit 20 mg Tetrakistriphenylphosphinpalladium versetzt. Man rührt 12 Stunden bei 100°C nach. Nach Abkühlen auf Raumtemp. wird zum Reaktionsgemisch Wasser gegeben und mit Ethylacetat extrahiert. Die vereinigten organischen Phasen werden über Magnesiumsulfat getrocknet, die Lösungsmittel im Vakuum entfernt und der Rückstand durch RP-HPLC gereinigt. Man erhält **68** als amorphen hellgelben Feststoff. C₂₇H₂₈N₂O₅ (460.53), MS(ESI): 461.20 (M + H⁺).

### Beispiel XLV

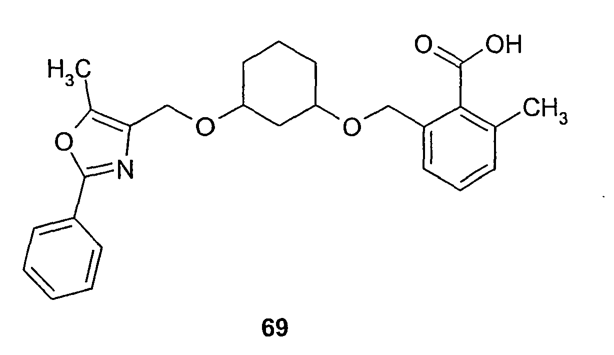

### 2-Methyl-6-[3-(5-methyl-2-phenyl-oxazol-4-ylmethoxy)-cyclohexyloxymethyl]-benzoesäure 69

Aus 2-(3-Hydroxy-cyclohexyloxymethyl)-6-methyl-benzoesäuremethylester und 2-Phenyl-4-iodmethyl-5-methyl-oxazol erhält man analog zu 50 das Produkt 69 mit dem Molekulargewicht 435.52 (C₂₆H₂₉NO₅), MS(ESI): 436.32 (M + H⁺).

### Beispiel XLVI

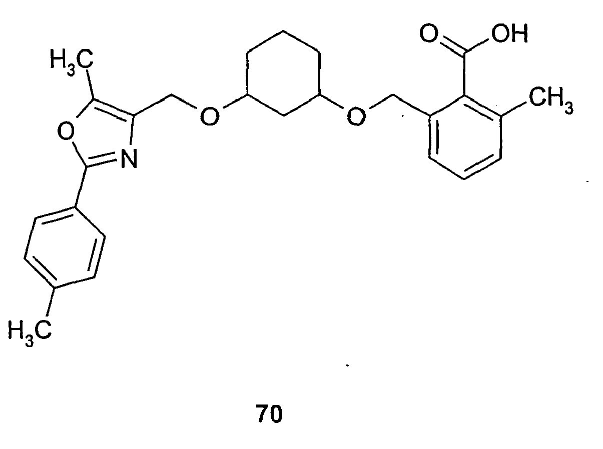

### 2-Methyl-6-[3-(5-methyl-2-p-tolyl-oxazol-4-ylmethoxy)-cyclohexyloxymethyl]-benzoesäure 70

Aus 2-(3-Hydroxy-cyclohexyloxymethyl)-6-methyl-benzoesäuremethylester und 2-(4-Methyl-phenyl)-4-iodmethyl-5-methyl-oxazol erhält man analog zu **50** das Produkt **70** mit dem Molekulargewicht 449.55 (C₂₇H₃₁NO₅), MS(ESI): 450.36 (M + H⁺).

### Beispiel XLVII

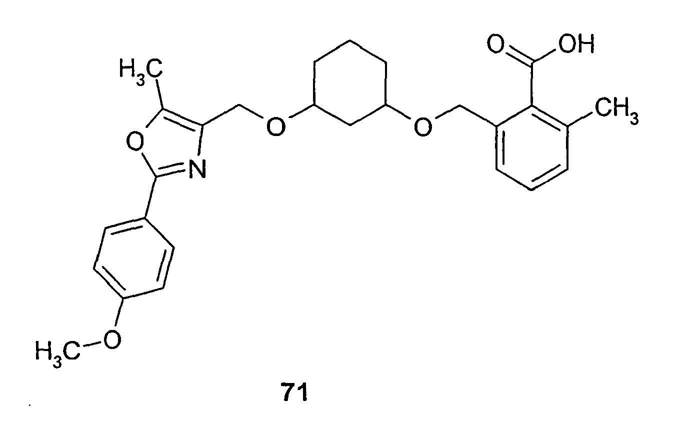

### 2-{3-[2-(4-Methoxy-phenyl)-5-methyl-oxazol-4-ylmethoxy]-cyclohexyloxymethyl}-6-methyl- benzoesäure 71

Aus 2-(3-Hydroxy-cyclohexyloxymethyl)-6-methyl-benzoesäuremethylester und 2-(4-Methoxy-phenyl)-4-iodmethyl-5-methyl-oxazol erhält man analog zu **50** das Produkt **71** mit dem Molekulargewicht 465.55 (C₂₇H₃₁NO₆), MS(ESI): 466.37(M + H⁺).

## Patentansprüche

1. Verbindungen der Formel I, 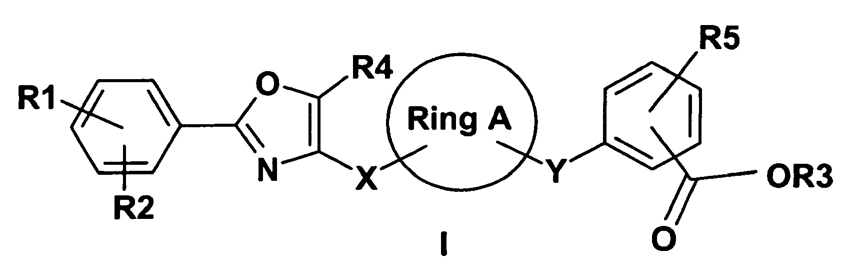 worin bedeuten
Ring A (C₃-C₈)-Cycloalkandiyl, (C₃-C₈)-Cycloalkendiyl, wobei in den Cycolalkandiyl- oder Cycloalkendiylringen ein oder mehrere Kohlenstoffatome durch Sauerstoffatome ersetzt sein können;
R1, R2, R4, R5 unabhängig voneinander H, F, Cl, Br, OH, NO₂, CF₃, OCF₃, (C₁-C₆)-Alkyl, O-(C₁-C₆)-Alkyl;
R3 H, (C₁-C₆)-Alkyl;
X (C₁-C₆)-Alkandiyl, wobei in der Alkandiylgruppe ein oder mehrere Kohlenstoffatome durch Sauerstoffatome ersetzt sein können;
Y (C₁-C₆)-Alkandiyl, wobei in der Alkandiylgruppe ein oder mehrere Kohlenstoffatome durch Sauerstoffatome ersetzt sein können;
sowie deren physiologisch verträgliche Salze.

2. Verbindungen der Formel I, gemäß Anspruch 1, **dadurch gekennzeichnet, daß** darin bedeuten
Ring A (C₃-C₈)-Cycloalkandiyl, (C₃-C₈)-Cycloalkendiyl, wobei in den Cycloalkandiyl- oder Cycloalkendiylringen ein oder mehrere Kohlenstoffatome durch Sauerstoffatome ersetzt sein können;
R1, R2, R4 unabhängig voneinander H, F, Cl, Br, OH, NO₂, CF₃, OCF₃, (C₁-C₆)-Alkyl, O-(C₁-C₆)-Alkyl;
R5 (C₁-C₆)-Alkyl;
R3 H, (C₁-C₆)-Alkyl;
X (C₁-C₆)-Alkandiyl, wobei in der Alkandiylgruppe ein oder mehrere Kohlenstoffatome durch Sauerstoffatome ersetzt sind;
Y (C₁-C₆)-Alkandiyl, wobei in der Alkandiylgruppe ein oder mehrere Kohlenstoffatome durch Sauerstoffatome ersetzt sein können;
sowie deren physiologisch verträgliche Salze.

3. Verbindungen der Formel I, gemäß Anspruch 1 oder 2, **dadurch gekennzeichnet, daß** darin bedeuten
Ring A (C₃-C₈)-Cycloalkandiyl, (C₃-C₈)-Cycloalkendiyl;
R1, R2 unabhängig voneinander H, F, Cl, Br, OH, NO₂, CF₃, OCF₃, (C₁-C₆)-Alkyl, O-(C₁-C₆)-Alkyl;
R3 H, (C₁-C₆)-Alkyl;
X (C₁-C₆)-Alkandiyl, wobei in der Alkandiylgruppe ein oder mehrere Kohlenstoffatome durch Sauerstoffatome ersetzt sind;
Y (C₁-C₆)-Alkandiyl, wobei in der Alkandiylgruppe ein oder mehrere Kohlenstoffatome durch Sauerstoffatome ersetzt sind;
sowie deren physiologisch verträgliche Salze.

4. Verbindungen der Formel I, gemäß einem oder mehreren der Ansprüche 1 bis 3 mit der Struktur Ia 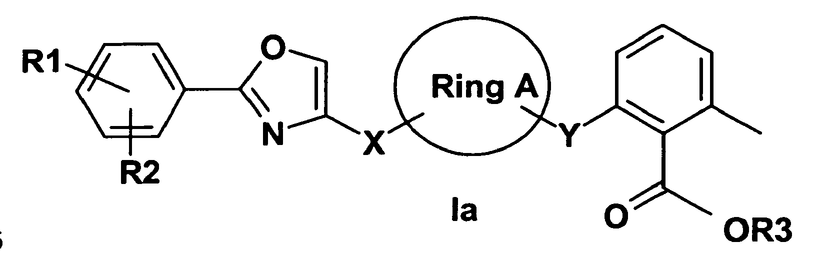
Ring A Cyclohexandiyl;
R1, R2 unabhängig voneinander H, F, Cl, Br, OH, NO₂, CF₃, OCF₃, (C₁-C₆)-Alkyl, O-(C₁-C₆)-Alkyl-;
R3 H, (C₁-C₆)-Alkyl;
X (C₁-C₆)-Alkandiyl, wobei in der Alkandiylgruppe ein oder mehrere Kohlenstoffatome durch Sauerstoffatome ersetzt sind;
Y C₁-C₆)-Alkandiyl, wobei in der Alkandiylgruppe ein oder mehrere Kohlenstoffatome durch Sauerstoffatome ersetzt sind;
sowie deren physiologisch verträgliche Salze.

5. Arzneimittel enthaltend eine oder mehrere der Verbindungen gemäß einem oder mehreren der Ansprüche 1 bis 4.

6. Arzneimittel enthaltend eine oder mehrere der Verbindungen gemäß einem oder mehreren der Ansprüche 1 bis 4 und ein oder mehrere Wirkstoffe.

7. Arzneimittel enthaltend eine oder mehrere der Verbindungen gemäß einem oder mehreren der Ansprüche 1 bis 4 und ein oder mehrere Lipid- oder Triglyceridsenkende Wirkstoffe

8. Verwendung der Verbindungen gemäß einem oder mehreren der Ansprüche 1 bis 4 zur Herstellung eines Medikamentes zur Behandlung von Lipidstoffwechselstörungen.

9. Verwendung der Verbindungen gemäß einem oder mehreren der Ansprüche 1 bis 4 zur Herstellung eines Medikamentes zur Behandlung von Typ II Diabetes.

10. Verwendung der Verbindungen gemäß einem oder mehreren der Ansprüche 1 bis 4 zur Herstellung eines Medikamentes zur Behandlung von Syndrom X.

11. Verwendung der Verbindungen gemäß einem oder mehreren der Ansprüche 1 bis 4 zur Herstellung eines Medikamentes zur Behandlung von gestörter Glucose Toleranz.

12. Verwendung der Verbindungen gemäß einem oder mehreren der Ansprüche 1 bis 4 zur Herstellung eines Medikamentes zur Behandlung von Essstörungen.

13. Verwendung der Verbindungen gemäß einem oder mehreren der Ansprüche 1 bis 4 zur Herstellung eines Medikamentes zur Behandlung von Obesitas.

14. Verwendung der Verbindungen gemäß einem oder mehreren der Ansprüche 1 bis 4 zur Herstellung eines Medikamentes zur Behandlung von Kardiomyopathie.

15. Verwendung der Verbindungen gemäß einem oder mehreren der Ansprüche 1 bis 4 zur Herstellung eines Medikamentes zur Behandlung von Herzinsuffizienz.

16. Verwendung der Verbindungen gemäß einem oder mehreren der Ansprüche 1 bis 4 zur Herstellung eines Medikamentes zur Behandlung von Osteoporose.

17. Verwendung der Verbindungen gemäß einem oder mehreren der Ansprüche 1 bis 4 zur Herstellung eines Medikamentes zur Behandlung von Atherosklerose.

18. Verwendung der Verbindungen gemäß einem oder mehreren der Ansprüche 1 bis 4 zur Herstellung eines Medikamentes zur Behandlung von Morbus Alzheimer.

19. Verwendung der Verbindungen gemäß einem oder mehreren der Ansprüche 1 bis 4 zur Herstellung eines Medikamentes zur Behandlung von Entzündungen.

20. Verwendung der Verbindungen gemäß einem oder mehreren der Ansprüche 1 bis 4 in Kombination mit mindestens einem weiteren Wirkstoff zur Herstellung eines Medikamentes zur Behandlung von Lipidstoffwechselstörungen.

21. Verwendung der Verbindungen gemäß einem oder mehreren der Ansprüche 1 bis 4 in Kombination mit mindestens einem weiteren Wirkstoff zur Herstellung eines Medikamentes zur Behandlung von Typ II Diabets.

22. Verwendung der Verbindungen gemäß einem oder mehreren der Ansprüche 1 bis 4 in Kombination mit mindestens einem weiteren Wirkstoff zur Herstellung eines Medikamentes zur Behandlung von Syndromen X.

23. Verfahren zur Herstellung eines Arzneimittels enthaltend eine oder mehrere der Verbindungen gemäß einem oder mehreren der Ansprüche 1 bis 4, **dadurch gekennzeichnet, daß** der Wirkstoff mit einem pharmazeutisch geeigneten Träger vermischt wird und diese Mischung in eine für die Verabreichung geeignete Form gebracht wird.

## Claims

1. A compound of the formula I 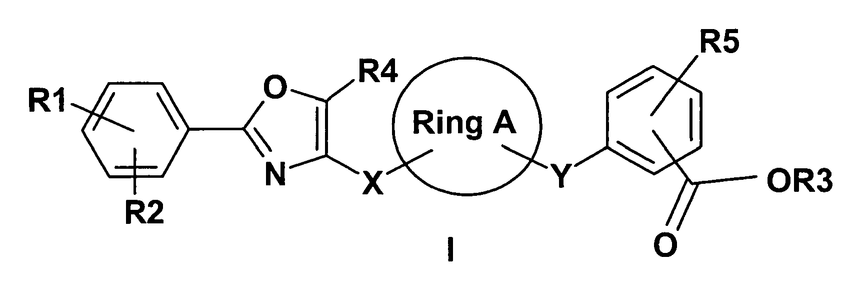 in which
Ring A is (C₃-C₈)-cycloalkanediyl or (C₃-C₈) - cycloalkenediyl, where in the cycloalkanediyl or cycloalkenediyl rings one or more carbon atoms may be replaced by oxygen atoms;
R1, R2, R4, R5 independently of one another are H, F, Cl, Br, OH, NO₂, CF₃, OCF₃, (C₁-C₆)-alkyl or O-(C₁-C₆)-alkyl;
R3 is H or (C₁-C₆)-alkyl;
X is (C₁-C₆)-alkanediyl, where in the alkanediyl group one or more carbon atoms may be replaced by oxygen atoms;
Y is (C₁-C₆)-alkanediyl, where in the alkanediyl group one or more carbon atoms may be replaced by oxygen atoms;
and its physiologically acceptable salts.

2. A compound of the formula I as claimed in claim 1, wherein
Ring A is (C₃-C₈)-cycloalkanediyl or (C₃-C₈)-cycloalkenediyl, where in the cycloalkanediyl or cycloalkenediyl rings one or more carbon atoms may be replaced by oxygen atoms;
R1, R2, R4 independently of one another are H, F, Cl, Br, OH, NO₂, CF₃, OCF₃, (C₁-C₆)-alkyl or O-(C₁-C₆)-alkyl;
R5 is (C₁-C₆)-alkyl;
R3 is H or (C₁-C₆)-alkyl;
X is (C₁-C₆)-alkanediyl, where in the alkanediyl group one or more carbon atoms are replaced by oxygen atoms;
Y is (C₁-C₆)-alkanediyl, where in the alkanediyl group one or more carbon atoms may be replaced by oxygen atoms;
and its physiologically acceptable salts.

3. A compound of the formula I as claimed in claim 1 or 2, wherein
Ring A is (C₃-C₈)-cycloalkanediyl or (C₃-C₈) - cycloalkenediyl;
R1, R2 independently of one another are H, F, Cl, Br, OH, NO₂, CF₃, OCF₃, (C₁-C₆) -alkyl or O- (C₁-C₆) -alkyl;
R3 is H or (C₁-C₆) -alkyl;
X is (C₁-C₆) -alkanediyl, where in the alkanediyl group one or more carbon atoms are replaced by oxygen atoms;
Y is (C₁-C₆)-alkanediyl, where in the alkanediyl group one or more carbon atoms are replaced by oxygen atoms;
and its physiologically acceptable salts.

4. A compound of the formula I as claimed in one or more of claims 1 to 3 having the structure Ia 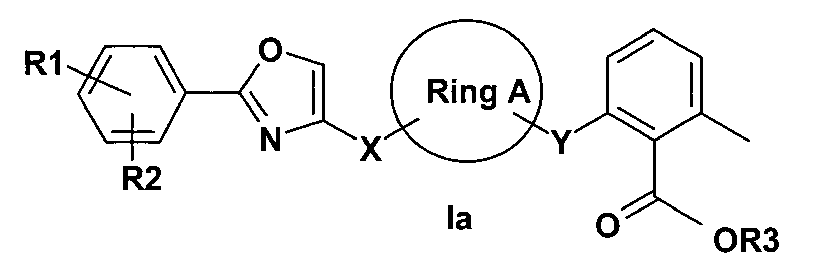
Ring A is cyclohexanediyl;
R1, R2 independently of one another are H, F, Cl, Br, OH, NO₂, CF₃, OCF₃, (C₁-C₆) -alkyl or O- (C₁-C₆)-alkyl;
R3 is H or (C₁-C₆)-alkyl;
X is (C₁-C₆)-alkanediyl, where in the alkanediyl group one or more carbon atoms are replaced by oxygen atoms;
Y is (C₁-C₆)-alkanediyl, where in the alkanediyl group one or more carbon atoms are replaced by oxygen atoms;
and its physiologically acceptable salts.

5. A pharmaceutical, comprising one or more compounds as claimed in one or more of claims 1 to 4.

6. A pharmaceutical comprising one or more compounds as claimed in one or more of claims 1 to 4 and one or more active compounds.

7. A pharmaceutical, comprising one or more compounds as claimed in one or more of claims 1 to 4 and one or more lipid- or triglyceride-lowering active compounds.

8. The use of the compounds as claimed in one or more of claims 1 to 4 for preparing a medicament for the treatment of disorders of the lipid metabolism.

9. The use of the compounds as claimed in one or more of claims 1 to 4 for preparing a medicament for the treatment of type II diabetes.

10. The use of the compounds as claimed in one or more of claims 1 to 4 for preparing a medicament for the treatment of syndrome X.

11. The use of the compounds as claimed in one or more of claims 1 to 4 for preparing a medicament for the treatment of disturbed glucose tolerance.

12. The use of the compounds as claimed in one or more of claims 1 to 4 for preparing a medicament for the treatment of eating disorders.

13. The use of the compounds as claimed in one or more of claims 1 to 4 for preparing a medicament for the treatment of obesity.

14. The use of the compounds as claimed in one or more of claims 1 to 4 for preparing a medicament for the treatment of cardiomyopathy.

15. The use of the compounds as claimed in one or more of claims 1 to 4 for preparing a medicament for the treatment of cardiac insufficiency.

16. The use of the compounds as claimed in one or more of claims 1 to 4 for preparing a medicament for the treatment of osteoporosis.

17. The use of the compounds as claimed in one or more of claims 1 to 4 for preparing a medicament for the treatment of atherosclerosis.

18. The use of the compounds as claimed in one or more of claims 1 to 4 for preparing a medicament for the treatment of Alzheimer's disease.

19. The use of the compounds as claimed in one or more of claims 1 to 4 for preparing a medicament for the treatment of inflammations.

20. The use of the compounds as claimed in one or more of claims 1 to 4 in combination with at least one further active compound for preparing a medicament for the treatment of disorders of the lipid metabolism.

21. The use of the compounds as claimed in one or more of claims 1 to 4 in combination with at least one further active compound for preparing a medicament for the treatment of type II diabetes.

22. The use of the compounds as claimed in one or more of claims 1 to 4 in combination with at least one further active compound for preparing a medicament for the treatment of syndrome X.

23. A process for preparing a pharmaceutical comprising one or more compounds as claimed in one or more of claims 1 to 4, which comprises mixing the active compound with a pharmaceutically suitable carrier and bringing this mixture into a form suitable for administration.

## Revendications

1. Composés de formule I, 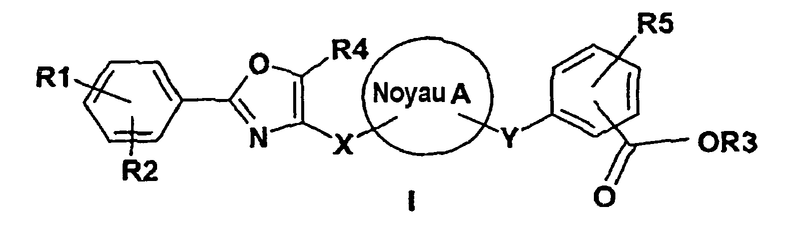 dans laquelle :
le Noyau A représente un groupe cycloalcandiyle (C₃-C₈) ou un groupe cycloalcendiyle (C₃-C₈), dans lesquels, dans les noyaux cycloalcandiyle ou cycloalcendiyle, un ou plusieurs atomes de carbone peuvent être remplacés par des atomes d'oxygène ;
R1, R2, R4, R5 représentent, indépendamment les uns des autres, un atome d'hydrogène, un atome de fluor, un atome de chlore, un atome de brome, un groupe OH, un groupe NO₂, un groupe CF₃, un groupe OCF₃, un groupe alkyle en (C₁-C₆) ou un groupe O-(C₁-C₆) alkyle ;
R3 représente un atome d'hydrogène ou un groupe alkyle en (C₁-C₆) ;
X représente un groupe alcandiyle en (C₁-C₆) dans lequel, dans le groupe alcandiyle, un ou plusieurs atomes de carbone peuvent être remplacés par des atomes d'oxygène ;
Y représente un groupe alcandiyle en (C₁-C₆) dans lequel, dans le groupe alcandiyle, un ou plusieurs atomes de carbone peuvent être remplacés par des atomes d'oxygène ;
ainsi que leurs sels physiologiquement acceptables.

2. Composés de formule I, selon la revendication 1, **caractérisés en ce que**, dans celle-ci :
le Noyau A représente un groupe cycloalcandiyle (C₃-C₈) ou un groupe cycloalcendiyle (C₃-C₈), dans lesquels, dans les noyaux cycloalcandiyle ou cycloalcendiyle, un ou plusieurs atomes de carbone peuvent être remplacés par des atomes d'oxygène ;
R1, R2, R4 représentent, indépendamment les uns des autres, un atome d'hydrogène, un atome de fluor, un atome de chlore, un atome de brome, un groupe OH, un groupe NO₂, un groupe CF₃, un groupe OCF₃, un groupe alkyle en (C₁-C₆) ou un groupe O- (C₁-C₆) alkyle ;
R5 représente un groupe alkyle en (C₁-C₆) ;
R3 représente un atome d'hydrogène ou un groupe alkyle en (C₁-C₆) ;
X représente un groupe alcandiyle en (C₁-C₆) dans lequel, dans le groupe alcandiyle, un ou plusieurs atomes de carbone peuvent être remplacés par des atomes d'oxygène ;
Y représente un groupe alcandiyle en (C₁-C₆) dans lequel, dans le groupe alcandiyle, un ou plusieurs atomes de carbone peuvent être remplacés par des atomes d'oxygène ;
ainsi que leurs sels physiologiquement acceptables.

3. Composés de formule I, selon la revendication 1 ou 2, **caractérisés en ce que** dans celle-ci :
le Noyau A représente un groupe cycloalcandiyle (C₃-C₈) ou un groupe cycloalcendiyle (C₃-C₈);
R1, R2 représentent, indépendamment l'un de l'autre, un atome d'hydrogène, un atome de fluor, un atome de chlore, un atome de brome, un groupe OH, un groupe NO₂ , un groupe CF₃, un groupe OCF₃, un groupe alkyle en (C₁-C₆) ou un groupe O- (C₁-C₆) alkyle ;
R3 représente un atome d'hydrogène ou un groupe alkyle en (C₁-C₆) ;
X représente un groupe alcandiyle en (C₁-C₆) dans lequel, dans le groupe alcandiyle, un ou plusieurs atomes de carbone peuvent être remplacés par des atomes d'oxygène ;
Y représente un groupe alcandiyle en (C₁-C₆) dans lequel, dans le groupe alcandiyle, un ou plusieurs atomes de carbone peuvent être remplacés par des atomes d'oxygène ;
ainsi que leurs sels physiologiquement acceptables.

4. Composés de formule I, selon l'une ou plusieurs des revendications 1 à 3, présentant la structure Ia 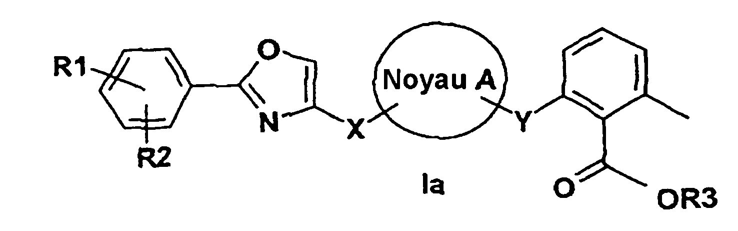 dans laquelle :
le Noyau A représente un groupe cyclohexandiyle ;
R1, R2 représentent, indépendamment l'un de l'autre, un atome d'hydrogène, un atome de fluor, un atome de chlore, un atome de brome, un groupe OH, un groupe NO₂, un groupe CF₃, un groupe OCF₃, un groupe alkyle en (C₁-C₆) ou un groupe O-(C₁-C₆) alkyle ;
R3 représente un atome d'hydrogène ou un groupe alkyle en (C₁-C₆) ;
X représente un groupe alcandiyle en (C₁-C₆) dans lequel, dans le groupe alcandiyle, un ou plusieurs atomes de carbone peuvent être remplacés par des atomes d'oxygène ;
Y représente un groupe alcandiyle en (C₁-C₆) dans lequel, dans le groupe alcandiyle, un ou plusieurs atomes de carbone peuvent être remplacés par des atomes d'oxygène ;
ainsi que leurs sels physiologiquement acceptables.

5. Agent pharmaceutique comprenant l'un ou plusieurs des composés selon l'une ou plusieurs des revendications 1 à 4.

6. Agent pharmaceutique comprenant l'un ou plusieurs des composés selon l'une ou plusieurs des revendications 1 à 4 et un ou plusieurs ingrédients actifs.

7. Agent pharmaceutique comprenant l'un ou plusieurs des composés selon l'une ou plusieurs des revendications 1 à 4 et un ou plusieurs ingrédients actifs hypolipémiants ou abaissant le taux de triglycérides.

8. Utilisation des composés selon l'une ou plusieurs des revendications 1 à 4 pour la production d'un médicament destiné au traitement de troubles du métabolisme lipidique.

9. Utilisation des composés selon l'une ou plusieurs des revendications 1 à 4 pour la production d'un médicament destiné au traitement du diabète du type II.

10. Utilisation des composés selon l'une ou plusieurs des revendications 1 à 4 pour la production d'un médicament destiné au traitement du syndrome X.

11. Utilisation des composés selon l'une ou plusieurs des revendications 1 à 4 pour la production d'un médicament destiné au traitement d'une tolérance perturbée du glucose.

12. Utilisation des composés selon l'une ou plusieurs des revendications 1 à 4 pour la production d'un médicament destiné au traitement de troubles de l'alimentation.

13. Utilisation des composés selon l'une ou plusieurs des revendications 1 à 4 pour la production d'un médicament destiné au traitement de l'obésité.

14. Utilisation des composés selon l'une ou plusieurs des revendications 1 à 4 pour la production d'un médicament destiné au traitement de la cardiomyopathie.

15. Utilisation des composés selon l'une ou plusieurs des revendications 1 à 4 pour la production d'un médicament destiné au traitement d'une insuffisance cardiaque.

16. Utilisation des composés selon l'une ou plusieurs des revendications 1 à 4 pour la production d'un médicament destiné au traitement de l'ostéoporose.

17. Utilisation des composés selon l'une ou plusieurs des revendications 1 à 4 pour la production d'un médicament destiné au traitement de l'athérosclérose.

18. Utilisation des composés selon l'une ou plusieurs des revendications 1 à 4 pour la production d'un médicament destiné au traitement de la maladie d'Alzheimer.

19. Utilisation des composés selon l'une ou plusieurs des revendications 1 à 4 pour la production d'un médicament destiné au traitement d'inflammations.

20. Utilisation des composés selon l'une ou plusieurs des revendications 1 à 4 en combinaison avec au moins un ingrédient actif supplémentaire pour la production d'un médicament destiné au traitement de troubles du métabolisme lipidique.

21. Utilisation des composés selon l'une ou plusieurs des revendications 1 à 4 en combinaison avec au moins un ingrédient actif supplémentaire pour la production d'un médicament destiné au traitement du diabète du type II.

22. Utilisation des composés selon l'une ou plusieurs des revendications 1 à 4 en combinaison avec au moins un ingrédient actif supplémentaire pour la production d'un médicament destiné au traitement du syndrome X.

23. Procédé de production d'un agent pharmaceutique comprenant l'un ou plusieurs des composés selon l'une ou plusieurs des revendications 1 à 4, **caractérisé en ce que** l'ingrédient actif est mélangé avec un support pharmaceutiquement approprié, et ce mélange est mis sous une forme appropriée pour l'administration.
